# EUROPEAN PATENT SPECIFICATION

(11) **EP 3 423 449 B1**
(45) Date of publication and mention of the grant of the patent: **07.04.2021**
(21) Application number: 17725779.7
(22) Date of filing: 03.03.2017
(51) Int. Cl.: C07D 407/04, C07D 409/06, C07D 409/14, C07D 407/14, A61K 31/4406, A61K 31/44, A61P 31/06

(54) **SUBSTITUTED AURONE ALKALOIDS AS ANTI-MYCOBACTERIAL AGENTS**
SUBSTITUIERTE AURONALKALOIDE ALS ANTIMYKOBAKTERIKA
ALCALOÏDES D'AURONE SUBSTITUÉS EN TANT QU'AGENTS ANTI-MYCOBACTÉRIENS

(30) Priority: 03.03.2016 IN 201611007477
(43) Date of publication of application: 09.01.2019
(73) Proprietor: Council of Scientific and Industrial Research, New Delhi 110001 (IN)
(72) Inventor: GUDUP, Satish Sonbarao, Jammu (IN); KUMAR, Sanjay, Jammu (IN); ARURI, Hari Prasad, Jammu (IN); SINGH, Umed, Jammu (IN); MUNAGALA, Gurunadham, Jammu (IN); YEMPALLA, Kushalava Reddy, Jammu (IN); SINGH, Samsher, Jammu (IN); KHAN, Inshad Ali, Jammu (IN); ASREY, Vishwakarma Ram, Jammu (IN); SINGH, Parvinder Pal, Jammu (IN)
(74) Representative: Kolster Oy Ab
(86) International application number: PCT/IN2017/050078
(87) International publication number: WO 2017/149551

(56) References cited:
- REYNOLDS R C ET AL: "High throughput screening of a library based on kinase inhibitor scaffolds against Mycobacterium tuberculosis H37Rv", TUBERCULOSIS, ELSEVIER, GB , vol. 92, no. 1 1 January 2012 (2012-01-01), pages 72-83, XP002729986, ISSN: 1472-9792, DOI: 10.1016/J.TUBE.2011.05.005 Retrieved from the Internet: URL:http://ac.els-cdn.com/S147297921100096 5/1-s2.0-S1472979211000965-main.pdf?_tid=d 2683624-543f-11e4-a4ba-00000aab0f6b&acdnat =1413359561_de1e85d49c56e40e5743d26b1e3a98 6d [retrieved on 2011-06-25]
- S. VADIVELAN ET AL: "Virtual Screening Studies to Design Potent CDK2-Cyclin A Inhibitors", JOURNAL OF CHEMICAL INFORMATION AND MODELING, vol. 47, no. 4, 1 July 2007 (2007-07-01), pages 1526-1535, XP055381265, US ISSN: 1549-9596, DOI: 10.1021/ci7000742 & S. Vadivelan ET AL: "Table 1s. Experimental and Predicted IC 50 values of 302 test set molecules", , 25 May 2007 (2007-05-25), XP055381406, Retrieved from the Internet: URL:http://pubs.acs.org/doi/suppl/10.1021/ ci7000742/suppl_file/ci7000742-file007.pdf [retrieved on 2017-06-14]
- TOMINAGA YUKIO ET AL: "General Model for Estimation of the Inhibition of Protein Kinases Using Monte Carlo Simulations", JOURNAL OF MEDICINAL CHEMISTRY, AMERICAN CHEMICAL SOCIETY, vol. 47, no. 10, 1 January 2004 (2004-01-01), pages 2534-2549, XP002658752, ISSN: 0022-2623, DOI: 10.1021/JM0304358
- JOSEPH SCHOEPFER ET AL: "Structure-Based Design and Synthesis of 2-Benzylidene-benzofuran-3-ones as Flavopiridol Mimics", JOURNAL OF MEDICINAL CHEMISTRY, vol. 45, no. 9, 1 April 2002 (2002-04-01), pages 1741-1747, XP055381267, ISSN: 0022-2623, DOI: 10.1021/jm0108348
- SIMS PETER A ET AL: "A Computational Model of Binding Thermodynamics: The Design of Cyclin-dependent Kinase 2 Inhibitors", JOURNAL OF MEDICINAL CHEMISTRY, AMERICAN CHEMICAL SOCIETY , vol. 46, no. 15 1 July 2003 (2003-07-01), pages 3314-3325, XP008146406, ISSN: 0022-2623, DOI: 10.1021/JM0205043 Retrieved from the Internet: URL:http://pubs.acs.org/journals/jmcmar/in dex.html [retrieved on 2003-06-12]

## Description

### FIELD OF THE INVENTION:

The present invention relates to the compounds of aurone alkaloid scaffold designed, synthesized and evaluated for their anti-tuberculosis. The present invention particularly relates to novel compounds of formula I, their method of preparations, and their use as drugs for treatment of tuberculosis.

### BACKGROUND OF THE INVENTION:

Tuberculosis remains a leading infectious cause of death worldwide and infects about one-third of the world's population. The World Health Organization (WHO) has estimated that if the present conditions remain unchanged, more than 30 million lives will be claimed by TB between 2000 and 2020. In 2012, an estimated 8.6 million people developed TB and 1.3 million died from the disease (including 320 000 deaths among HIV-positive people). TB has also been declared as a global health emergency because of the increase in secondary infections and/or co-infection in cancer and immunocompromised patients (such as those infected with human immunodeficiency virus). The existing lengthy TB therapy and emergence of multidrug resistant TB (MDR-TB) and extensively drug resistant TB (XDR-TB), BemerMelchior, P.; Bryskier, A.; Drugeon, H. B. J. Antimicrob. Chemother. 2000, 46, 571; Abubaker, J.; Schraufnagel, D. J. Am. Med. Assoc. 2000, 283, 54; Dye. C.; Scheele, S.; Dolin, P.; Pathania, V.; Raviglione, M. C. J. Am. Med. Assoc. 1999, 282, 677] necessitates the development of new and potent anti-tuberculosis agents.

Aurones are known for various biological activities including anti-tubercular activity, however, regarding aurone alkaloid, there is one report (Joseph Schoepfer, Heinz Fretz, Bhabatosh Chaudhuri, Lionel Muller, Egge Seeber, Laurent Meijer, Olivier Lozach, Eric Vangrevelinghe, and Pascal Furet. J. Med. Chem. 2002, 45, 1741), where a series of aurone aurones alkaloids were synthesized and screened CDK inhibitory activity with reference to anti-cancer potential. R.C. Reynolds et al., in "High throughput screening of a library based on kinase inhibitor scaffolds against Mycobacterium tuberculosis H37Rv" Tuberculosis, 92, 72-83, 2012 reported high throughput screening results for targeted library of approximately 26000 compounds that was designed based on kinase inhibitor scaffolds and known kinase binding sites. The selection of scaffolds is based on enzymatic screening against specific kinase or ATP binding sites in M. tuberculosis with the in vitro activity against whole bacteria.

The emergence of drug-resistant in tuberculosis and problems associated with co-infection with other diseases, necessitates the need for the discovery and development of novel chemical entities for tuberculosis. In this direction, aurone alkaloids is representing new scaffold with interesting pharmacological profile against tuberculosis, which may serve as future therapeutics for tuberculosis.

### OBJECTIVE OF THE INVENTION:

The main objective of the present invention is to provide novel aurone alkaloids for the treatment of tuberculosis.

### SUMMARY OF THE INVENTION:

Accordingly, the present invention provides a compound of formula I pharmaceutically acceptable salts thereof, 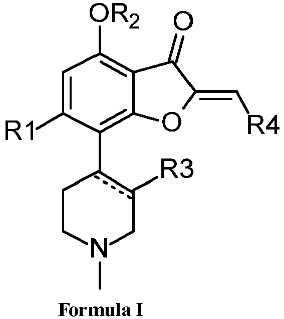 Wherein
R₁ is selected from the group consisting of H, OH, alkoxy,
R₂ is selected from the group consisting of CF₃, alkyl,
----Represents an optional second carbon - carbon bond,
R₃ is selected from the group consisting of H, OH,
R₄ is selected from the group consisting of alkyl, substituted alkyl, styryl, substituted styryl, aryl, substituted aryl, biaryl, substituted biaryl, napthyl, anthracenyl, substituted or unsubstituted heterocycle or heteroaryl and heterocycle or heteroaryl is selected from the group consisting of furanyl, thiophenyl, perazinyl, morpholinyl, piperidyl, pyridyl, triazolyl, triazinyl, pyrimidinyl, pyridazinyl, oxazolyl, benzofuranyl, pyrrolyl, imidazoyl, thiazoyl, quinolinyl, isoquinolinyl, benzooxazolyl, and benzothiazolyl and further substituted aryl, substituted styryl, substituted vinyl, substituted biaryl and substituted heteroaryl or heterocycle;
wherein the substitution ranges from 1 to 4 and the substituents are independently selected from the group consisting of F, Cl, I, NR_{I1}R_{I2}, CF₃, OCF₃, OR_{I3}, NO₂, alkyl chain from C₁ to C₁₄,
R_{I1}, R_{I2} and R_{I3} are each independently selected from the group consisting of H, alkyl, substituted alkyl phenyl, substituted phenyl, propargyl;

In an embodiment of the invention wherein representative compounds comprising:
(*E*)-2-(2-Chlorobenzylidene)-4,6-dimethoxy-7-(1-methylpiperidin-4-yl)benzofuran-3(2*H*)-one (Compound **I1**, Table 1)
(*E*)-2-(2-Fluorobenzylidene)-4,6-dimethoxy-7-(1-methylpiperidin-4-yl)benzofuran-3(2*H*)-one (Compound **I2,** Table 1)
   (*Z*)-4,6-Dimethoxy-2-(2-methylbenzylidene)-7-(1-methylpiperidin-4-yl)benzofuran-3(2*H*) -one (Compound **I3,** Table 1)
   (*E*)-4,6-Dimethoxy-2-(2-methoxybenzylidene)-7-(1-methylpiperidin-4-yl)benzofuran-3(2*H*)-one (Compound **I4,** Table 1)
   (*E*)-2-(2-Iodobenzylidene)-4,6-dimethoxy-7-(1-methylpiperidin-4-yl)benzofuran-3(2*H*)-one (Compound **I5,** Table 1)
   (*Z*)-2-(4-Fluorobenzylidene)-4,6-dimethoxy-7 -(1-methylpiperidin-4-yl)benzofuran-3(2*H*)-one(Compound **I6,** Table 1)
   (*Z*)-4,6-Dimethoxy-2-(4-methoxybenzylidene)-7-(1-methylpiperidin-4-yl)benzofuran-3(2*H*)-one (Compound I**7,** Table 1)
   (*Z*)-2-[4-(Dimethylamino)benzylidene]-4,6-dimethoxy-7-(1-methylpiperidin-4-yl) benzo furan-3(2*H*)-one (Compound **I8,** Table 1)
   (*Z*)-4,6-Dimethoxy-7-(1-methylpiperidin-4-yl)-2-[4-(prop-2-yn-1-yloxy)benzylidene] benzofuran-3(2*H*)-one (Compound **I9,** Table 1)
   (*Z*)-2-(3-Bromobenzylidene)-4,6-dimethoxy-7-(1-methylpiperidin-4-yl)benzofuran-3(2*H*)-one(Compound **I10,** Table 1)
   (*Z*)-2-(3-Bromo-4-fluorobenzylidene)-4,6-dimethoxy-7-(1-methylpiperidin-4-yl)benzo furan-3(2*H*)-one (Compound **I11,** Table 1)
   (*Z*)-2-(2,6-Dichlorobenzylidene)-4,6-dimethoxy-7-(1-methylpiperidin-4-yl)benzofuran 3(2*H*)-one (Compound I**12,** Table 2)
   (*Z*)-2-[2,6-Bis(trifluoromethyl)benzylidene]-4,6-dimethoxy-7-(1-methylpiperidin-4yl)benzofuran-3(2*H*)-one(Compound **I13,** Table 2)
   (*Z*)-2-Benzylidene-4,6-dimethoxy-7-(1-methylpiperidin-4-yl)benzofuran-3(2*H*)-one (Compound **I14,** Table 2)
   (*E*)-4,6-Dimethoxy-7-(1-methylpiperidin-4-yl)-2-(naphthalen-1-ylmethylene)benzofuran-3(2*H*)-one (Compound **I15,** Table 2)
   (*E*)-2-(Anthracen-9-ylmethylene)-4,6-dimethoxy-7-(1-methylpiperidin-4-yl)benzofuran-3(2*H*)-one (Compound **I16,** Table 2)
   (*E*)-4,6-dimethoxy-7-(1-methylpiperidin-4-yl)-2-(thiophen-2-ylmethylene)benzofuran-3(2*H*)-one (Compound **I17,** Table 2)
   (*Z*)-2-(Furan-2-ylmethylene)-4,6-dimethoxy-7-(1-methylpiperidin-4-yl)benzofuran-3(2*H*)-one (Compound **I18,** Table 2)
   (*E*)-4,6-Dimethoxy-7-(1-methylpiperidin-4-yl)-2-(pyridin-3-ylmethylene)benzofuran-3(2*H*)-one (Compound **I19,** Table 2)
   (*Z*)-4,6-Dimethoxy-7-(1-methylpiperidin-4-yl)-2-(2,4,5-trimethoxybenzylidene)benzo furan-3(2*H*)-one (Compound **I20**, Table 2)
   (*Z*)-4,6-Dimethoxy-7-(1-methylpiperidin-4-yl)-2-(2,4,6-trimethoxybenzylidene)benzo furan-3(2*H*)-one (Compound **I21,** Table 2)
   (*E*)-4,6-Dimethoxy-7-(1-methylpiperidin-4-yl)-2-((Z)-3-phenylallylidene)benzofuran-3(2*H*)-one (Compound **I22**, Table 2)
   (*Z*)-4,6-Dimethoxy-7-(1-methylpiperidin-4-yl)-2-((Z)-3-(o-tolyl)allylidene)benzofuran-3(2*H*)-one (Compound **I23**, Table 2)
   (*E*)-4,6-Dihydroxy-7-(1-methylpiperidin-4-yl)-2-(thiophen-2-ylmethylene)benzofuran-3(2*H*)-one (Compound **I24,** Table 2)
   (*E*)-2-(2-Chlorobenzylidene)-4-ethoxy-7-(1-methylpiperidin-4-yl)benzofuran-3(2*H*)-one (Compound **I25**, Table 2)
   (*Z*)-2-(4-Bromobenzylidene)-4-ethoxy-7-(1-methy|piperidin-4-yl)benzofuran-3(2*H*)-one (Compound **I26**, Table 3)
   (*E*)-2-(2-Chlorobenzylidene)-4,6-dimethoxy-7-(1-methyl-1,2,3,6-tetrahydropyridin-4-yl)benzofuran-3(2*H*)-one (Compound **I27**, Table 3)
   (*E*)-2-(2-Fluorobenzylidene)-4,6-dimethoxy-7-(1-methyl-1,2,3,6-tetrahydropyridin-4-yl)benzofuran-3(2*H*)-one (Compound **I28**, Table 3)
   (*E*)-4,6-Dimethoxy-7-(1-methyl-1,2,3,6-tetrahydropyridin-4-yl)-2-(2-methylbenzylidene) benzofuran-3(2*H*)-one (Compound 129, Table 3)
   (*Z*)-2-(4-Fluorobenzylidene)-4,6-dimethoxy-7-(1-methyl-1,2,3,6-tetrahydropyridin-4-yl)benzofuran-3(2*H*)-one (Compound **I30,** Table 3)
   (*Z*)-2-(2,6-Dichlorobenzylidene)-4,6-dimethoxy-7-(1-methyl-1,2,3,6-tetrahydropyridin-4-yl)benzofuran-3(2*H*)-one (Compound **I31,** Table 3)
   (Z)-2-Benzylidene-4,6-dimethoxy-7-(1-methyl-1 ,2,3,6-tetrahydropyridin-4-yl)benzofur an-3(2*H*)-one (Compound **I32,** Table 3)
   (*E*)-4,6-Dimethoxy-7-(1 -methyl-1,2,3,6-tetrahydropyridin-4-yl)-2-(naphthalen-1-ylme-thylene)benzofuran-3(2*H*)-one (Compound **I33,** Table 3)
   (*Z*)-2-(Furan-2-ylmethylene)-4,6-dimethoxy-7-(1-methyl-1,2,3,6-tetrahydropyridin-4-yl)benzofuran-3(2*H*)-one (Compound **I34,** Table 3)
   (*E*)-4,6-Dimethoxy-7-(1-methyl-1,2,3,6-tetrahydropyridin-4-yl)-2-(thiophen-2-ylmethy lene)benzofuran-3(2*H*)-one (Compound **I35,** Table 3)
   (*E*)-2-(2-Chlorobenzylidene)-7-(3-hydroxy-1-methylpiperidin-4-yl)-4,6-dimethoxy benzofuran-3(2*H*)-one (Compound **I36,** Table 3)
   (*Z*)-2-(Furan-2-ylmethylene)-4-hydroxy-6-methoxy-7-(1-methylpiperidin-4-yl)benzo furan-3(2*H*)-one (Compound **I37,** Table 3)
   (*E*)-4-Ethoxy-2-(2-fluorobenzylidene)-7-(1-methyl-1,2,3,6-tetrahydropyridin-4-yl)ben zofuran-3(2*H*)-one (Compound **I38,** Table 3)
   (Z)-2-(4-Bromobenzylidene)-4-ethoxy-7-(1-methyl-1,2,3,6-tetrahydropyridin-4-yl)benzo - furan-3(2*H*)-one (Compound **I39,** Table 3)
      In another embodiment of the invention wherein the compounds are useful in treatment of tuberculosis.
      In still another embodiment of the invention wherein the pharmaceutically acceptable salts are salts of an acid selected from the group consisting of hydrochloric acid, sulphuric acid, phosphoric acid, acetic acid, citric acid, oxalic acid, malonic acid, salyilic acid, malic acid, fumaric acid, succinic acid, ascorbic acid, maleic acid, methanesulfonic acid, and isoethonic acids.
      Accordingly the present invention also provides a process for preparation of compounds of general formula formula 1 wherein the process steps comprising;
      (i) reacting compound of formula 2 or 3 with an aldehyde compound in presence of a base in protic solvent at a temperature ranging between 20 to 100°C for a period in the range of 2 to 12 hr to obtain compound of formula 6 or 5 respectively,
      (ii) reacting compound of formula 5 or 6 with Hg(OAc)₂ in presence of base at a temperature in the range of 60 to 150 °C for a period in the range of 1 to 12 hr to give compound of general formula 1.
      In another embodiment of the invention wherein the aldehyde is selected from a group consisting of alkyl, substituted alkyl, styryl, substituted styryl, aryl, substituted aryl, biaryl, substituted biaryl, napthyl, anthracenyl, substituted or unsubstituted heterocycles or heteroaryl and heterocycle or heteroaryl is selected from the group consisting of furanyl, thiophenyl, perazinyl, morpholinyl, piperidyl, pyridyl, triazolyl, triazinyl, pyrimidinyl, pyridazinyl, oxazolyl, benzofuranyl, pyrrolyl, imidazoyl, thiazoyl, quinolinyl, isoquinolinyl, benzooxazolyl, benzothiazolyl and further substituted aryl, substituted styryl, substituted vinyl, substituted biaryl and substituted hetrocycle or heteroaryl wherein the substitution ranges from 1 to 4 and the substituents are independently selected from the group consisting of F, Cl, I, NR_{I1}R_{I2}, CF₃, OCF₃, OR_{I3}, NO₂, alkyl chain from C₁ to C₁₄,
      R_{I1}, R_{I2} and R_{I3} are each independently selected from the group consisting of H, alkyl, substituted alkyl phenyl, substituted phenyl.

In an embodiment of the invention wherein the base used is selected from a group consisting of KOH, NaOH, K₂CO₃, Cs₂CO₃, pyridine, triethylamine.

In another embodiment of the invention wherein the protic solvent used is selected from a group consisting of methanol, ethanol, water.

A pharmaceutical composition comprising the compound of formula 1, optionally along with a pharmaceutically acceptable carrier, salt, excipients or diluents,

In an embodiment of invention of general formula I, are useful in prevention of or therapy for treatment of tuberculosis.

In yet another embodiment of the invention of general formula I, wherein said compound exhibits an *in-vitro* anti-tuberculosis activity against H₃₇Rv Mycobacterium tuberculosis with MIC values in the range of 1 to 64 µg/ml.

### Brief description of the drawings:

Fig 1 shows the structure of general formula I.
Fig 2 shows the synthetic scheme (Scheme 1) for the synthesis of compounds **I1-I26**.
Fig 3 shows the synthetic scheme (Scheme 2) for the synthesis of the compounds **I27-I39.**
Table 1, Table 2 and Table 3 shows the structures of compounds **I1** to **I39.**
Table 4 shows the Anti-tuberculosis activity against H₃₇Rv of representative compounds **I1** to I**39** shown in Table 1, 2 & 3.

### LIST OF ABBREVATIONS:

- ATCC: : American type culture collection
- AcOH: : acetic acid
- CFU: : colony forming units
- DCM: : dichloromethane
- d: : doublet
- dd: : doublet of doublet
- Et: : ethyl
- ESI: : electron spray ionisation
- FCS: : fetal calf serum
- H₃₇Rv: : a well characterised virulent strain of *Mycobacterium tuberculosis*
- h: : hours
- IC₅₀: : half maximal inhibitory concentration
- *J*: : coupling constant
- MIC: : minimum inhibitory concentration
- MS: : mass spectrometry
- ml: : millilitre
- MHz: : mega hertz
- m: : multiplet
- MDR-TB: : Multi drug resistant tuberculosis
- Me: : methyl
- min: : minutes
- m/z: : mass to charge ratio
- MTB: : *Mycobacterium tuberculosis*
- NMP: : N-methylpyrrolidinone
- Rif^{R}: : rifampicin resistant tuberculosis
- RPMI: : rosewell park memorial institute medium
- R_{f}: : retention factor
- s: : singlet
- TFA: : trifluoroacetic acid
- TLC: : thin layer chromatography
- TB: : Tuberculosis
- TDR-TB: : Total drug resistant tuberculosis
- *t*: : triplet
- *tert*: : tertiary
- WHO: : world health organization
- XDR-TB: : Extensive drug resistant tuberculosis
- µg: : microgram
- ¹H NMR: : proton nuclear magnetic resonance

### DETAILED DESCRIPTION OF THE INVENTION:

The present invention relates to novel aurone alkaloids, their use as drugs for treatment of tuberculosis.

The present invention describes a compound having general structure of formula I

In a first aspect, the present invention pertains to a compound having a general structure of formula I 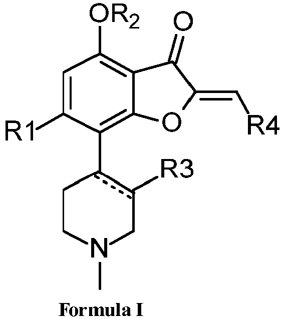 Wherein
R₁ is selected from the group consisting of H, OH, alkoxy
R₂ is selected from the group consisting of CF₃, alkyl
---- Represents an optional second carbon - carbon bond
R₃ is selected from the group consisting of H, OH
R₄ is selected from the group consisting of alkyl, substituted alkyl, styryl, substituted styryl, aryl, substituted aryl, biaryl, substituted biaryl, napthyl, anthracenyl, substituted or unsubstituted heterocycle or heteroaryl and heterocycle or heteroaryl group is selected from the group consisting of furanyl, thiophenyl, perazinyl, morpholinyl, piperidyl, pyridyl, triazolyl, triazinyl, pyrimidinyl, pyridazinyl, oxazolyl, benzofuranyl, pyrrolyl, imidazoyl, thiazoyl, quinolinyl, isoquinolinyl, benzooxazolyl, and benzothiazolyl and further substituted aryl, substituted styryl, substituted vinyl, substituted biaryl and substituted heteroaryl or heterocycle wherein the substitution ranges from 1 to 4 and substituents are independently selected from the group consisting of F, Cl, **I,** NR_{I1}R_{I2}, CF₃, OCF₃, OR_{I3}, NO₂, alkyl chain from C₁ to C₁₄.
R_{I1}, R_{I2} and R_{I3} are each independently selected from the group consisting of H, alkyl, substituted alkyl phenyl, substituted phenyl;

The compound of general formula **I,** exhibits an *in vitro* anti-tuberculosis activity against H₃₇Rv *Mycobacterium tuberculosis* with the MIC values in the range of 1 to 64 µg/ml

In the present invention, the pharmaceutically acceptable salts of general formula **I,** are salts of an acid selected from the group consisting of hydrochloric acid, sulfuric acid, phosphoric acid, acetic acid, citric acid, oxalic acid, malonic acid, salyilic acid, malic acid, fumaric acid, succinic acid, ascorbic acid, maleic acid, methanesulfonic acid and isoethonic acid or salts of a base selected from the group consisting of potassium carbonate, sodium hydroxide, potassium hydroxide, ammonia, triethylamine and triethanolamine.

### Synthetic schemes:

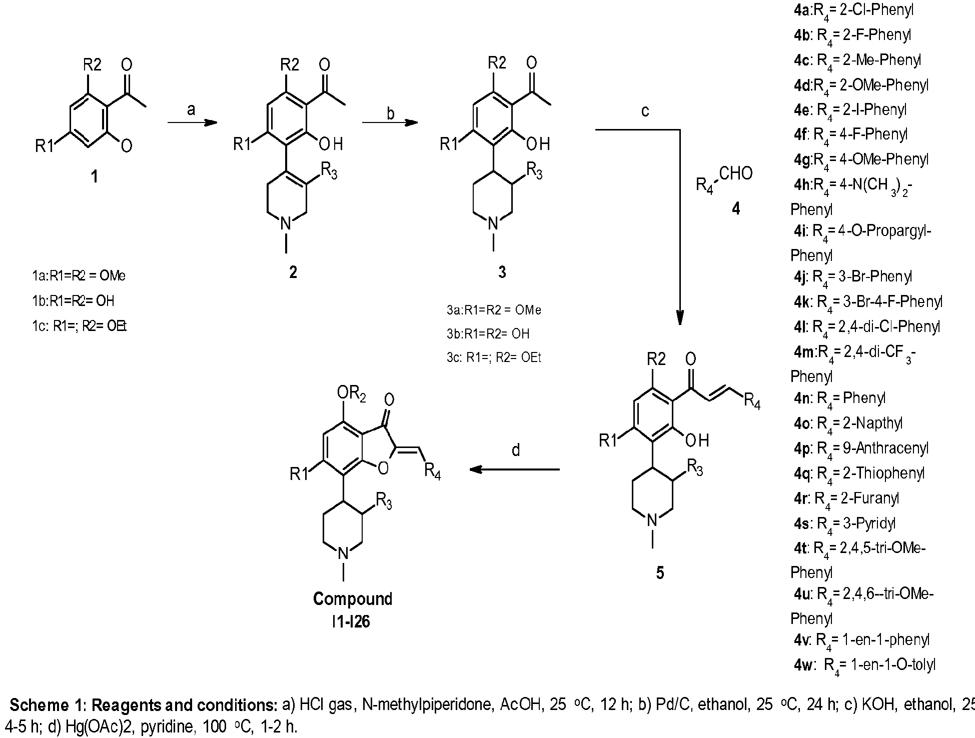

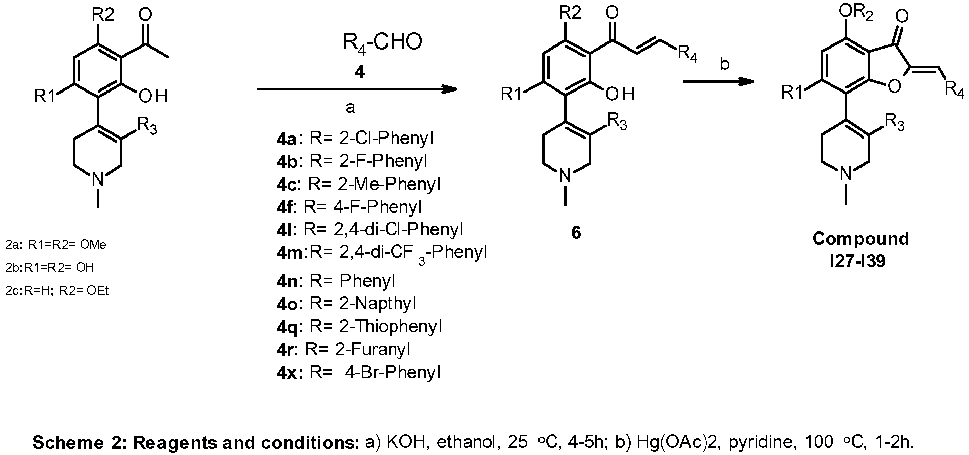

### Examples:

### Example-1 :

### General procedure for the preparation of compounds of formula 2:

HCl gas was bubbled in a mixture of compound **1** (102 mmol) and 1-methyl-4-piperidone (122 mmol) in acetic acid (100 ml) for 1h. The reaction mixture was stirred at room temperature for 24h, the solvent was removed under reduced pressure, and ice cold water (100 ml) is added to the residue. The aqueous solution is extracted with ether (100 ml x 2), and the aqueous portion is basified by addition of 40% sodium hydroxide solution. The precipitate obtained is filtered, washed with water and dried. Recrystallization from petroleum ether to obtained the pure compounds of formula **2.**

### Example-2:

### General procedure for the preparation of compounds of formula 3:

Compounds of formula **2** (57.6 mmol) was hydrogenated in ethanol (220 ml) over Pd/C (10%) at normal pressure and room temperature for 20 h. The reaction mixture was filtered over celite, the solvent evaporated under reduced pressure, and recrystallization of the residue from ethanol afforded the hydrochloride salt of formula **3** as a white solid.

### Example-3:

### General procedure for the preparation of compounds of formula 5 and 6:

To a solution of compounds of formula **2** or **3** (1 mmol) with substituted benzaldehyde **4** (1.2 mmol) in ethanol (10 ml) was added potassium hydroxide (1.5 mmol) at room temperature. After being stirred at 25 °C for 3-4 h, solvent evaporated under reduced pressure the crude mixture was partitioned between water and ethyl acetate, separate the organic layer washed with brine solution and dried over anhydrous sodium sulphate and evaporated under vaccuo. The crude products were purified by column chromatography with dichloromethane methanol as eluting solvents.

In addition to the above Example **3** (condition 1), the following reaction conditions as shown in tables were also used for the preparation of compounds of formula **5** and **6.**

| **Reactant-1** | **Reactant-2** | **Reaction conditions** | | **Yield** | **Products** |
|---|---|---|---|---|---|
| **Compound 3** | **Compound 4** | **Condition 1** | **KOH, Ethanol, 25°C, 3-4h.** | **70-85%** | **Compound 5** |
| | | Condition 2 | NaOH, Water, 25°C, 3-4h. | 60-75% | |
| | | Condition 3 | K₂CO₃, Methanol, 80°C, 3-4h. | 70-75% | |
| | | Condition 4 | Cs₂CO₃, Ethanol, 80 °C, 12h. | 50-65% | |

| Reactants | | Reaction conditions | | Yield | Products |
|---|---|---|---|---|---|
| **Compound 2** | **Compound 4** | **Condition 1** | **KOH, Ethanol, 25°C, 3-4h.** | **70-85%** | **Compound 6** |
| | | Condition 2 | NaOH, Water, 25°C, 3-4h. | 60-75% | |
| | | Condition 3 | K₂CO₃, Methanol, 80°C, 3-4h. | 70-75% | |
| | | Condition 4 | Cs₂CO₃, Ethanol, 80 °C, 12h. | 50-65% | |

### Example-4:

### General procedure for the preparation of compounds I1 to I39

To a solution of compounds of formula **5** or **6** (1 mmol) in pyridine (3 ml) was added mercury acetate (1.2 mmol) at room temperature (25-30 °C). The reaction mixture was stirred at 100 °C for 1 h, and the solvent was evaporated under vaccuo. The crude products were purified by column chromatography with dichloromethane methanol as eluting solvents to obtain the pure compounds .

In addition to the above Example **4** (condition 1), the following reaction conditions as shown in tables were also used for the preparation of compounds **I1** to **I39**.

### (E)-2-(2-Chlorobenzylidene)-4,6-dimethoxy-7-(1-methylpiperidin-4-yl)benzofuran-3(2H)-one (I1, Fig 2, Table-1):

| **Reactants** | **Reaction conditions** | | **Yield** | **Products** |
|---|---|---|---|---|
| **Compounds 5** | **Condition 1** | **Pyridine, mercury acetate, 100 °C**, **1h**. | **50-85%** | **Compounds I1-I26** |
| | Condition 2 | Et₃N, mercury acetate, 100°C, 3h, | 30-55% | |
| **Compounds 6** | **Condition 1** | **Pyridine, mercury acetate, 100 °C, 1h.** | **50-85%** | **Compounds 127-139** |
| | Condition 2 | Et₃N, mercury acetate, 100°C, 3h, | 30-55% | |

TLC (DCM: MeOH 9:1): *R_{f}*= 0.5; Yellow solid; ¹H NMR (400 MHz, CDCl₃) δ 8.16 (d, *J* = 8.0 Hz, 1H), 7.42 (d, *J* = 6.5 Hz, 1H), 7.32 (d, *J =* 7.8 Hz, 1H), 7.24 - 7.19 (m, 1H), 7.17 (s, 1H), 6.14 (s, 1H), 3.99 (s, 3H), 3.93 (s, 3H), 3.55 (d, *J =* 8.1 Hz, 2H), 3.18 (t*, J =* 10.0 Hz, 1H), 2.80 - 2.69 (m, 7H), 1.82 (d, *J =* 10.3 Hz, 2H); HRMS (ESI): calcd for C₂₃H₂₅ClNO₄ [M+H⁺] 414.1467, found 414.1483.

### (E)-2-(2-Fluorobenzylidene)-4,6-dimethoxy-7-(1-methylpiperidin-4-yl)benzofuran-3(2H)-one (12, Fig 2, Table-1):

TLC (DCM: MeOH 9:1): *R_{f}*= 0.5; yellow solid; ¹H NMR (400 MHz, CDCl₃) δ 8.25 (d, *J =* 7.2 Hz, 1H), 7.35 - 7.29 (m, 2H), 7.12 (dd, *J* = 7.8, 6.9 Hz, 1H), 7.04 (s, 1H),6.14(s, 1H), 4.00 (s, 3H), 3.94 (s, 3H), 3.17-3.14 (m, 3H), 2.55 *(d, J=* 18.0 Hz, 2H), 2.47 (s,3H), 2.24 (s, 2H), 1.71 (d*, J* = 13.2 Hz, 2H); HRMS (ESI): calcd for C₂₃H₂₅FNO₄ [M+H⁺] 398.1762, found 398.1763.

### (Z)-4,6-Dimethoxy-2-(2-methylbenzylidene)-7-(1-methylpiperidin-4-yl)benzofuran-3(2H)-one (I3, Fig 2, Table-1):

TLC (DCM: MeOH 9:1): R_{F=} 0.5; yellow solid; ¹H NMR (400 MHz, CDCl₃) δ 8.16 (d, *J* = 7.7 Hz, 1H), 7.33 (t, *J =* 6.9 Hz, 1H), 7.26 (d, *J =* 7.5 Hz, 2H), 6.97 (s, 1H), 6.14 (s, 1H), 4.00 (s, 3H), 3.95 (s, 3H), 3.21 - 3.04 (m, 3H), 2.50 (s, 8H), 2.30 (s, 2H), 1.70 (d, *J =* 12.9 Hz, 2H). Hz, 2H); HRMS (ESI): Calcd for C₂₄H₂₈NO₄ [M+H⁺]:394.2013 Found 394.2015

### (E)-4,6-Dimethoxy-2-(2-methoxybenzylidene)-7-(1-methylpiperidin-4-yl)benzofuran-3(2H)-one (I4, Fig 2, Table-1):

TLC (DCM: MeOH 9:1): *R_{f}*= 0.5; Yellow solid ; ¹H NMR (400 MHz, CDCl₃) δ 8.21 (d, *J* = 7.5 Hz, 1H), 7.35 (t*, J =* 7.9 Hz, 1H), 7.31 (s, 1H), 7.09 (t*, J =* 7.6 Hz, 1H), 6.93 (d*, J* = 8.3 Hz, 1H), 6.14 (s, 1H), 4.00 (s, 3H), 3.96 (s, 3H), 3.90 (s, 3H), 3.30 - 3.07 (m, 3H), 2.73 - 2.49 (m, 5H), 2.47 - 2.30 (m, 2H), 1.73 (d, *J =* 13.4 Hz, 2H); HRMS (ESI): calcd for C₂₄H₂₇NO₄ [M+H⁺]:410.1962, found 410.1965.

### (E)-2-(2-Iodobenzylidene)-4,6-dimethoxy-7-(1-methylpiperidin-4-yl)benzofuran-3(2H)-one(I5, Fig2, Table-1):

TLC (DCM: MeOH 9:1): R*_{f}*= 0.5; Yellow solid; ¹H NMR (400 MHz, CDCl₃) δ 8.02 - 7.73 (m, 1H), 7.90 - 7.77 (m, 1H), 7.66 (dd, *J* =13.4, 7.0 Hz, 1H), 7.43 (d, *J =,* 7.7 Hz, 1H), 7.07 - 6.96 (m, 1H), 6.01 (s, 1H), 3.96 (s, 3H), 3.91 (s, 3H), 3.48-3.36 (m, 3H), 2.97 - 2.87 (m, 2H), 2.75 (s, 3H), 2.62 (d, *J =* 5.1 Hz, 2H), 1.63 (d, *J =* 13.4 Hz, 2H); HRMS (ESI): calcd for C₂₃H₂₅INO₄ [M+H⁺]:506.0823, found 506.0820..

### (Z)-2-(4-Fluorobenzylidene)-4,6-dimethoxy-7-(1-methylpiperidin-4-yl)benzofuran-3(2H)-one (I6, Fig 2, Table-1):

TLC (DCM: MeOH 9:1): *R_{f}*= 0.5; Yellow solid; ¹H NMR (400 MHz, CDCl₃) δ 8.09 (dd, *J* = 8.4, 5.8 Hz, 1H), 7.85 (dd, *J* = 8.6, 5.5 Hz, 1H), 7.15 (dd, *J* = 16.6, 8.0 Hz, 1H), 7.06 - 6.91 (m, 2H), 6.72 (s, 1H), 6.14 (s, 1H), 4.00 (s, 3H), 3.93 (s, 3H), 3.38 - 3.07 (m, 3H), 2.67 - 2.32 (m, 7H), 1.76 (d, *J* = 12.5 Hz, 2H); HRMS (ESI): calcd for C₂₃H₂₅FNO₄ [M+H⁺]:398.1762, found 398.1740.

### (Z)-4,6-Dimethoxy-2-(2-methoxybenzylidene)-7-(1-methylpiperidin-4-yl)benzofuran-3(2H)-one (I7, Fig 2, Table-1):

TLC (DCM: MeOH 9:1): *R_{f}* = 0.5; Yellow solid; ¹H NMR (400 MHz, CDCl₃) δ 7.81 (d, *J* = 8.8 Hz, 2H), 7.02 (d, *J =* 8.8 Hz, 1H), 6.98 - 6.83 (m, 1H), 6.74 (s, 1H), 6.13 (s, 1H), 3.99 (s, 3H), 3.94 (s, 3H), 3.85 (s, 3H), 3.53 - 3.12 (m, 3H), 2.77 - 2.57 (m, 7H), 1.80 (d, *J=* 13.3 Hz, 2H); HRMS (ESI): calcd for C₂₄H₂₈NO₅ [M+H⁺]:410.1962, found 410.1944.

### (Z)-2-(4-(Dimethylamino)benzylidine)-4,6-dimethoxy-7-methylpiperidin-4-yl) benzofuran-3(2H)-one (18, Fig 2, Table-1):

TLC (DCM: MeOH 9:1): *R_{f}*= 0.5; Orange solid; ¹H NMR (400 MHz, MeOD) δ 7.64 (d, *J=* 8 Hz, 2H), 6.72 (d, *J=* 8 Hz, 2H), 6.54 (s, 1H), 6.23 (s, 1H), 3.87-3.86 (d, *J=* 4 Hz, 6H), 3.21 (s, 1H), 3.00 (d, *J=* 12 Hz, 2H), 2.95 (s, 6H), 2.32 (s, 3H), 2.18 (t, *J=* 12.0 Hz, 2H), 1.63 (d, *J=* 12.6 Hz, 2H); HRMS (ESI): calcd for C₂₅H₃₁N₂O₄ [M+H⁺]:423.2284, found 422.2205.

### (Z)-4,6-Dimethoxy-7-(1-methylpiperidin-4-yl)-2-(4-(prop-2-yn-1-yloxy)benzylidene) benzofuran-3(2H)-one (19, Fig 2, Table-1):

TLC (DCM: MeOH 9:1): *R_{f}* = 0.5; Yellow solid; ¹H NMR (400 MHz, CDCl₃) δ 7.85 (d, *J* = 8.0 Hz, 2H), 7.07 (d, *J =* 8.0 Hz, 2H), 6.74 (s, 1H), 6.14 (s, 1H), 4.77(d, *J* =4 Hz, 2H), 4.00 (s, 3H), 3.95 (s, 3H), 3.25-3.13 (m, 3H), 2.56 (t, *J* =4 Hz ,2H), 2.52 (brs, 5H), 2.28 (t *J* = 8Hz,2H) 1.74 - 1.71 (m, 2H); HRMS (ESI): calcd for C₂₆H₂₈NO₅ [M+H⁺]:434.1962, found 434.1937.

### (Z)-2-(3-Bromobenzylidene)-4,6-dimethoxy-7-(1-methylpiperidin-4-yl)benzofuran-3(2H)-one (110, Fig 2, Table-1):

TLC (DCM: MeOH 9:1): *R_{f}*= 0.5; Yellow solid; ¹H NMR (400 MHz, CDC13) δ 8.16 (d, *J*=7 Hz, 1H), 7.64 (d, *J* = 7.2 Hz, 1H), 7.55 (d, *J =* 7.7 Hz, 1H), 7.38 - 7.24 (m, 1H), 6.67 (s, 1H), 6.13 (s, 1H), 3.99 (s, 3H), 3.90 (s, 3H), 3.45 (d, *J=* 9.9 Hz, 2H), 3.19 (s, 1H), 2.65 (t, *J* = 21.7 Hz, 7H), 1.82 (d, *J* = 12.6 Hz,2H); HRMS (ESI): calcd for C₂₃H₂₅BrNO₄[M+H⁺]:458.0961, found 458.0946.

### (Z)-2-(3-Bromo-4-fluorobenzylidene)-4,6-dimethoxy-7-(1-methylpiperidin-4-yl)benzo furan-3(2H)-one (I11, Fig 2, Table-2):

TLC (DCM: MeOH 9:1): *R_{f}*= 0.5; Yellow solid; ¹H NMR (400 MHz, CDCl₃) δ 8.20 (s, 1H), 7.88 (d*, J*= 8.0 Hz, 1H), 7.07 (d, *J=* 8.0 Hz, 1H), 6.65 (s, 1H), 6.13 (s, 1H), 3.99 (s, 3H), 3.96 (s, 3H), 3.05 (d*, J*= 12 Hz, 3H), 2.46-2.44 (m, 5H), 2.12 (t*, J=* 11.7 Hz, 2H), 1.81 - 1.66 (m, 2H); HRMS (ESI): calcd for C₂₃H₂₄BrFNO₄ [M+H⁺]:476.0873, found 478.0911.

### (Z)-2-(2,6-Dichlorobenzylidene)-4,6-dimethoxy-7-(1-methylpiperidin-4-yl)benzofuran-3(2H)-one (112, Fig 2, Table-2):

TLC (DCM: MeOH 9:1): R_{F=} 0.5; yellow solid. ; ¹H NMR (400 MHz, CDCl₃) δ 7.39 (d, *J* = 8.0 Hz, 1H), 7.36 - 7.19 (m, 2H), 6.81 (s, 1H), 6.13 (s, 1H), 4.01 (s, 3H), 3.97 (s, 3H), 3.44-3.08 (m, 3H), 2.76 (d, *J =* 16 Hz, 4H), 2.62 (s, 3H), 1.70 (d, *J* = 12 Hz, 2H). HRMS (ESI): calcd for C₂₃H₂₄Cl₂NO₄ [M+H⁺]:477.0873, found 478.0911.

### (Z)-2-(2,6-Bis(trifluoromethyl)benzylidene)-4,6-dimethoxy-7-(1-methylpiperidin-4-yl)benzofuran-3(2H)-one (113, Fig 2, Table-2):

TLC (DCM: MeOH 9:1): *R_{f}*= 0.5; Yellow solid; ¹H NMR (400 MHz, CDCl₃) δ 8.56 (s, 1H), 8.34 (d*, J* = 10.5 Hz, 1H), 7.88 (dd, *J* = 28.2, 13.5 Hz, 1H), 6.78 (s, 1H), 6.14 (s, 1H), 4.00 (s, 3H), 3.86 (s, 3H), 3.21 (s, 1H), 2.90 (s, 5H), 2.81 (s, 2H), 1.89 (d, *J* = 13.4 Hz, 2H); HRMS (ESI): calcd for C₂₅H₂₃F₆NO₄ [M+H⁺]:516.1610, found 516.1611.

### (Z) 2-Benzylidene-4,6-dimethoxy-7-(1-methylpiperidin-4-yl)benzofuran-3(2H)-one(I14, Fig 2, Table-2):

TLC (DCM: MeOH 9:1): R_{F=} 0.5; Red solid ¹H NMR (400 MHz, CDCl₃) δ 7.83 (d, *J* = 7.4 Hz, 2H), 7.49 (d, *J* = 6.8 Hz, 1H), 7.40 (d, *J* = 6.8 Hz, 2H), 6.76 (s, 1H), 6.14 (s, 1H), 4.02 (s, 3H), 3.89 (s, 3H), 3.15 (d*, J* = 12 Hz, 3H), 2.45 - 2.34 (m, 5H), 2.12 (t*, J =* 11.7 Hz, 2H), 1.81 - 1.66 (m, 2H) HRMS (ESI-TOF)

### (E)-4,6-Dimethoxy-7-(1-methylpiperidin-4-yl)-2-(naphthalen-1-ylmethylene)benzofuran -3(2H)-one (115, Fig 2, Table-2):

TLC (DCM: MeOH 9:1): *R_{f}*= 0.5; Yellow solid; ¹H NMR (400 MHz, CDCl₃) δ 8.34 - 8.29 (m, 2H), 7.88 (dd*, J=* 8.2, 3.1 Hz, 2H), 7.65 - 7.56 (m, 3H), 7.54 (s, 1H), 6.16 (s, 1H), 4.02 (s, 3H), 3.96 (s, 3H), 3.26-3.07 (m, 3H), 2.59 (d, *J =* 17.7 Hz, 2H), 2.52 (s, 3H), 1.74 (d, *J =* 11.8 Hz, 2H); HRMS (ESI): calcd for C₂₇H₂₈NO₄ [M+H⁺]:430.2018, found 430.2012.

### (E)-2-(Anthracen-9-ylmethylene)-4,6-dimethoxy-7-(1-methylpiperidin-4-yl)benzofuran-3(2H)-one (116, Fig 2, Table-2):

TLC (DCM: MeOH 9:1): *R_{f}*= 0.5; Red solid; ¹H NMR (400 MHz, MeOD) δ 8.51 (s, 1H), 8.07 - 7.97 (m, 4H), 7.59 (s, 1H), 7.44 (dd, *J* = 4 Hz, 3H), 6.32 (s, 1H), 3.94 (s, 3H), 3.88 (s, 3H), 3.22 (m, 3H), 2.69 (d, *J=* 12 Hz, 2H), 2.11 (t*, J=* 12 Hz, 2H), 2.01 (s, 3H), 1.79 (t*, J=* 12 Hz, 2H); HRMS (ESI): calcd for C₃₁H₃₀NO₄ [M+H⁺]:480.2175, found 480.2178.

### (E)-4,6-Dimethoxy-7-(1-methylpiperidin-4-yl)-2-(thiophen-2-ylmethylene)benzofuran-3(2H)-one (117, Fig 2, Table-2):

TLC (DCM: MeOH 9:1): *R_{f}*= 0.5; Yellow solid; ¹H NMR (400 MHz, DMSO) δ 7.92 (d, *J* = 4.9 Hz, 1H), 7.64 (s, 1H), 7.27 - 7.16 (m, 1H), 7.10 (s, 1H), 6.45 (s, 1H), 3.97 (s, 2H), 3.94 (s, 2H), 3.06 - 2.90 (m, 3H), 2.51 (s, 3H), 2.42 - 2.30 (m, 2H), 2.25 (s, 3H), 1.54 (d, *J* = 11.9 Hz, 2H); HRMS (ESI): calcd for C₂₁H₂₄NO₄S [M+H⁺]:480.2175, found 480.2178.

### (Z)-2-(Furan-2-ylmethylene)-4,6-dimethoxy-7-(1-methylpiperidin-4-yl)benzofuran-3(2H)-one (118, Fig 2, Table-2):

TLC (DCM: MeOH 9:1): *R_{f}*= 0.5; Yellow solid; ¹H NMR (400 MHz, DMSO) δ 7.85 (d, *J* = 1.4 Hz, 1H), 7.06 (d*, J*= 3.4 Hz, 1H), 6.70 (dd, *J*= 3.0, 1.7 Hz, 1H), 6.55 (s, 1H), 6.38 (s, 1H), 3.90 (s, 3H), 3.87 (s, 3H), 2.98 - 2.81 (m, 3H), 2.51 - 2.37 (m, 5H), 2.19 (d, *J=* 12.4 Hz, 2H), 1.89 (d, *J* = 12.3 Hz, 2H). HRMS (ESI): calcd for C₂₁H₂₄NO₅[M+H⁺]:370.1654, found 370.1651.

### (E)-4,6-Dimethoxy-7-(1-methylpiperidin-4-yl)-2-(pyridin-3-ylmethylene)benzofuran-3(2H)-one (119, Fig 2, Table-2):

TLC (DCM: MeOH 9:1): *R_{f}*= 0.5; Yellow solid; ¹H NMR (400 MHz, CDCl₃) δ 8.56 (d, *J* = 4.4 Hz, 1H), 7.72 (t, *J=* 8.4 Hz, 1H), 7.50 (d, *J =* 8.1 Hz, 1H), 7.22 (d, *J =* 10.5 Hz, 1H), 7.20 (s,1H) ,5.94 (s, 1H), 3.96 (s, 3H), 3.86 (s, 3H), 3.47 (d, *J =* 15.7 Hz,3H), 2.79 (s, 2H), 2.32 (s, 3H), 2.02 (s, 2H). 1.8 (d, 2H).

### (Z)-4,6-Dimethoxy-7-(1-methylpiperidin-4-yl)-2-(2,4,5-trimethoxybenzylidene)benzo furan -3(2H)-one (120, Fig 2, Table-2):

TLC (DCM: MeOH 9:1): *R_{f}*= 0.5; Yellow solid; ¹H NMR (400 MHz, CDCl₃) δ 7.84 (s, 1H), 7.29 (s, 1H), 6.53 (s, 1H), 6.13 (s, 1H), 4.01 (s, 3H), 3.99 (s, 3H), 3.96 (s, 3H), 3.94 (s, 3H), 3.90 (s, 3H), 3.09 (dd, *J* = 11.6, 2.2 Hz, 3H), 2.60 - 2.46 (m, 2H), 2.37 (s, 5H), 1.66 (d, *J*= 11.7 Hz, 2H); HRMS (ESI): calcd for C₂₆H₃₂NO₇ [M+H⁺]:470.2179, found 470.2181.

### (Z)-4,6-Dimethoxy-7-(1-methylpiperidin-4-yl)-2-(2,4,6-trimethoxybenzylidene) benzofuran-3(2H)-one (121, Fig 2, Table-2):

TLC (DCM: MeOH 9:1): *R_{f}*= 0.5; Yellow solid; ¹H NMR (400 MHz, CDCl₃) 6.94 (s, 1H), 6.17 (s, 2H), 6.08 (s, 1H), 3.97 (s, 3H), 3.91 (s, 3H), 3.87 (s, 3H), 3.86 (s, 6H), 2.98 (t*, J =* 13.3 Hz, 3H), 2.44 - 2.34 (m, 2H), 2.30 (s, 3H), 2.01 (t*, J =* 11.4 Hz,2H), 1.56 (d*, J* = 12.3 Hz, 2H); HRMS (ESI): Calcd for C₂₆H₃₂NO₇ [M+H⁺]:470.2179, found 470.2172.

### (E)-4,6-Dimethoxy-7-(1-methylpiperidin-4-yl)-2-((E)-3-phenylallylidene)benzofuran-3(2H)-one (122, Fig 2, Table-2):

TLC (DCM: MeOH 9:1): *R_{f}*= 0.5; Yellow solid; ¹H NMR (400 MHz, CDCl₃) δ 7.59 (d, *J* = 7.6 Hz, 1H), 7.37 (dd, *J* = 15.6, 8.0 Hz, 2H), 6.98 (d, *J =* 15.5 Hz, 2H), 6.67 (d, *J =* 11.6 Hz, 1H), 6.11 (s, 1H), 3.98 (s, 3H), 3.92 (s, 3H), 3.45 (d, *J=* 10.1 Hz, 3H), 2.78 - 2.57 (m, 7H), 1.76 (d, *J* = 14.6 Hz, 2H); HRMS (ESI): calcd for C₂₅H₂₈NO₄ [M+H⁺]:406.2018, found 406.1992.

### (Z)-4,6-Dimethoxy-7-(1-methylpiperidin-4-yl)-2-((Z)-3-(0-tolyl)allylidene)benzofuran-3(2H)-one (123, Fig 2, Table-2):

TLC (DCM: MeOH 9:1): *R_{f}*= 0.5; Yellow solid; ¹H NMR (400 MHz, CDCl₃) δ 7.43 - 7.32 (m, 4H), 7.26 (s, 1H), 7.02 (s, 1H), 6.56 (s, 1H), 6.13 (s, 1H), 3.99 (s, 3H), 3.94 (s, 3H), 3.22 - 3.01 (m, 3H), 2.52 (d, *J =* 16.2 Hz, 2H), 2.47 (s, 3H), 2.43 (d, *J =* 1.1 Hz, 3H), 2.31 (d, *J =* 11.6 Hz, 2H), 1.70 (d, *J =* 10.3 Hz, 2H); HRMS (ESI): calcd for C₂₆H₃₀NO₄ [M+H⁺]: 420.2175, found 420.2165.

### (E)-4,6-Dihydroxy-7-(1-methylpiperidin-4-yl)-2-(thiophen-2-ylmethylene)benzofuran-3(2H)-one (124, Fig2, Table-2):

TLC (DCM: MeOH 9:1): *R_{f}*= 0.5; orange solid; ¹H NMR (400 MHz, MeOD) δ 7.64 (s, 1H), 7.41 (s, 1H), 7.08 (s, 1H), 6.92 (s, 1H), 6.02 (s, 1H), 3.57 (d, *J* = 11.2 Hz, 3H), 3.14 (d*, J* = 12.2 Hz, 2H), 2.85 (s, 3H), 2.59 (d*, J* = 12.5 Hz, 2H), 1.94 (d, *J =* 11.5 Hz, 2H); HRMS (ESI): calcd for C₁₉H₂₀NO₄ [M+H⁺]:358.1113, found 358.1110.

### (E)-2-(2-Chlorobenzylidene)-4-ethoxy-7-(1-methylpiperidin-4-yl)benzofuran-3(2H)-one (125, Fig 2, Table-3):

TLC (DCM: MeOH 9:1): *R_{f}*= 0.5; Yellow solid; ¹H NMR (400 MHz, CDCl₃) δ 8.24 (d, *J* = 9.1 Hz, 1H), 7.50 (dd, *J* = 16.9, 8.2 Hz, 2H), 7.41 (t*, J =* 7.6 Hz, 1H), 7.35 (s, 1H), 7.32 (d*, J =* 7.7 Hz, 1H), 6.63 (d, *J* = 8.6 Hz, 2H), 4.24 (q, *J* = 7.0 Hz, 1H), 3.39 (s, 1H), 3.23 (d, *J* = 12.3 Hz, 1H), 3.03 - 2.94 (m, 1H), 2.51 (s, 3H), 2.40 (d, *J* = 14.6 Hz, 2H), 2.01 (d, *J* = 7.0 Hz, 4H), 1.51 (t, *J* = 7.0 Hz, 3H).

### (Z)-2-(4-Bromobenzylidene)-4-ethoxy-7-(1-methylpiperidin-4-yl) benzofuran-3(2H)-one (126, Fig 2, Table-3):

TLC (DCM: MeOH 9:1): R_{F=} 0.5; yellow solid; ¹H NMR (400 MHz, MeOD) δ 7.75 (d, *J* = 8.5 Hz, 2H), 7.63 (d, *J =* 8.5 Hz, 2H), 7.51 (d, *J =* 8.5 Hz, 1H), 6.77 (s, 1H), 6.64 (d*, J =* 8.6 Hz, 1H), 4.25 (q, *J* = 7.0 Hz, 2H), 3.36 (dt*, J =* 3.2, 1.6 Hz, 1H), 3.21 (d, *J =* 12.5 Hz, 1H), 3.02 (d, *J =* 4.7 Hz, 1H), 2.51 (s, 3H), 2.45 (d, *J =* 17.9 Hz, 2H), 2.02 (d, *J* = 7.5 Hz, 4H), 1.51 (t, *J* = 7.0 Hz, 3H).

### (E)-2-(2-Chlorobenzylidene)-4,6-dimethoxy-7-(1-methyl-1,2,3,6-tetrahydropyridin-4-yl)benzofuran-3(2H)-one (I27, Fig3, Table-3):

TLC (DCM: MeOH 9:1): *R_{f}*= 0.5; Yellow solid; ¹H NMR (400 MHz, CDCl₃) δ 8.26 (d, *J* = 9.0 Hz, 1H), 7.44 (d, *J =* 8.4 Hz, 1H), 7.34 (d, *J =* 7.0 Hz, 1H), 7.29 (d, *J =* 11.6 Hz, 1H), 7.19 (s, 1H), 6.15 (s, 1H), 5.87 (s, 1H), 4.02 (s, 3H), 3.92 (s, 3H), 3.26 (d, *J =* 2.4 Hz, 2H), 2.78 (t, *J* = 5.6 Hz, 2H), 2.55 (s, 2H), 2.51 (s, 3H); HRMS (ESI): calcd for C₂₃H₂₃ClNO₄ [M+H⁺]:412.1316, found 412.1309.

### (E)-2-(2-Fluorobenzylidene)-4,6-dimethoxy-7-(1-methyl-1,2,3,6-tetrahydropyridin-4-yl)benzofuran-3(2H)-one (128, Fig 3, Table-3):

TLC (DCM: MeOH 9:1): *R_{f}*= 0.5; Yellow solid; ¹H NMR (400 MHz, CDCl₃) δ 7.29 (d, *J* = 8.2 Hz, 2H), 7.10 (s, 2H), 7.02 (s, 1H),6.14 (s, 1H), 5.76 (s, 1H), 4.02 (s, 3H), 3.93 (s, 3H), 3.28 (s, 2H), 2.81 (t, *J =* 5.5 Hz, 2H), 2.41(s, 5H); HRMS (ESI): calcd for C₂₃H₂₃FNO₄ [M+H⁺]:396.1611, found 396.1607.

### (E)-4,6-Dimethoxy-7-(1-methyl-1,2,3,6-tetrahydropyridin-4-yl)-2-(2-methylbenzylidene) benzofuran-3(2H)-one (129, Fig 3, Table-3):

TLC (DCM: MeOH 9:1): *R_{f}*= 0.5; Yellow solid; ¹H NMR (500 MHz, CDCl₃) δ 7.92 (s, 1H), 7.37 - 7.31 (m, 2H), 7.21 (d, *J =* 4.3 Hz, 1H), 5.96 (s, 1H), 5.55 (s, 1H), 3.93 (s, 3H), 3.81 (s, 3H), 3.36 (s, 2H), 2.91 (s, 2H), 2.62 (s, 3H)), 2.60 (s, 5H). HRMS (ESI): Calcd for C₂₄H₂₆NO₄ [M+H⁺]:392.1862, found 392.1800.

### (Z)-2-(4-Fluorobenzylidene)-4,6-dimethoxy-7-(1-methyl-1,2,3,6-tetrahydropyridin-4-yl)benzofuran-3(2H)-one (130, Fig 3, Table-3):

TLC (DCM: MeOH 9:1): *R_{f}*= 0.5; Yellow solid; ¹H NMR (400 MHz, CDCl₃) δ 7.83 (dd, *J* = 8.6, 5.5 Hz, 2H), 7.13 (t, *J* = 8.7 Hz, 2H), 6.72 (s, 1H), 6.15 (s, 1H), 5.88 (s, 1H), 4.02 (s, 3H), 3.93 (s, 3H), 3.49 (s, 2H), 3.04 (t, *J =* 5.8 Hz, 2H), 2.66 (s, 5H); HRMS (ESI): calcd for C₂₃H₂₃FNO₄ [M+H⁺]:396.1611, found 396.1607.

### (Z)-2-(2,6-Dichlorobenzylidene)-4,6-dimethoxy-7-(1-methyl-1,2,3,6-tetrahydropyridin-4-yl)benzofuran-3(2H)-one (131, Fig 3, Table-3):

TLC (DCM: MeOH 9:1): *R_{f}*= 0.5; Yellow solid; ¹H NMR(400 MHz, CDCl₃) δ 7.36 (d, *J =* 8.0 Hz, 2H), 7.23 (t*, J =* 8.0 Hz, 1H), 6.79 (s, 1H), 6.14 (s, 1H), 5.75 (s, 1H), 4.02 (s, 3H), 3.92 (s, 3H), 3.28 (s, 2H), 2.86 (t*, J =* 5.5 Hz, 2H), 2.48 (s, 5H); HRMS (ESI): calcd for C₂₃H₂₂Cl₂NO₄ [M+H⁺]:446.0926, found 446.0926.

### (Z)-2-Benzylidene-4,6-dimethoxy-7-(1-methyl-1,2,3,6-tetrahydropyridin-4-yl)benzo furan-3(2H)-one (132, Fig 3, Table-3):

TLC (DCM: MeOH 9:1): *R_{f}*= 0.5; Yellow solid; ¹H NMR (400 MHz, CDCl₃) δ 7.83 (d, *J* = 7.4 Hz, 2H), 7.49 (d, *J* = 6.8 Hz, 1H), 7.40 (d, *J =* 6.8 Hz, 2H), 6.76 (s, 1H), 6.14 (s, 1H), 5.92 (s, 1H), 4.02 (s, 3H), 3.89 (s, 3H), 3.24 - 3.13 (m, 2H), 2.78 (s, 5H), 2.69 (s, 2H).

### (E)-4,6-Dimethoxy-7-(1-methyl-1,2,3,6-tetrahydropyridin-4-yl)-2-(naphthalen-1-ylmethylene)benzofuran-3(2H)-one(133, Fig 3, Table-3):

TLC (DCM: MeOH 9:1): *R_{f}*= 0.5; Yellow solid; ¹H NMR (400 MHz, CDCl₃) δ 8.36 (dd, *J* = 14.0, 7.9 Hz, 2H), 7.88 (d, *J =* 7.7 Hz, 2H), 7.70 - 7.44 (m, 4H), 6.16 (s, 1H), 5.90 (s, 1H), 4.04 (s, 3H), 3.93 (s, 3H), 3.31 (s, 2H), 2.84 (s, 2H), 2.59 (s, 2H), 2.53 (s, 3H); HRMS (ESI): calcd for C₂₇H₂₅NO₄ [M+H⁺]:428.1784, found 428.1868.

### (Z)-2-(Furan-2-ylmethylene)-4,6-dimethoxy-7-(1-methyl-1,2,3,6-tetrahydropyridin-4-yl)benzofuran-3(2H)-one (134, Fig 3, Table-3):

TLC (DCM: MeOH 9:1): *R_{f}*= 0.5; Yellow solid; ¹H NMR (400 MHz, CDCl₃) δ 7.48 (s, 1H), 6.92 (d, *J =* 3.4 Hz, 1H), 6.70 (s, 1H), 6.51 (s, 1H), 6.07 (s, 1H), 5.80 (s, 1H), 3.94 (s, 3H), 3.85 (s, 3H), 3.39 (d, *J =* 2.4 Hz, 2H), 2.93 (t, *J* = 5.7 Hz, 2H), 2.67 - 2.46 (m, 5H). HRMS (ESI): calcd for C₂₁H₂₂NO₅ [M+H⁺]:368.1498, found 368.1491.

### (E)-4,6-Dimethoxy-7-(1-methyl-1,2,3,6-tetrahydropyridin-4-yl)-2-(thiophen-2-ylmethylene)benzofuran-3(2H)-one (135, Fig 3, Table-3):

TLC (DCM: MeOH 9:1): *R_{f}*= 0.5; Yellow solid; ¹H NMR (400 MHz, CDCl₃) δ 7.56 (d, *J* = 5.1 Hz, 1H), 7.42 (d*, J* = 3.5 Hz, 1H), 7.11 (dd, *J =* 5.1, 3.7 Hz, 1H), 7.06 (s, 1H), 6.15 (s, 1H), 5.93 (s, 1H), 4.02 (s, 3H), 3.93 (s, 3H), 3.41 (d, *J =* 2.7 Hz, 2H), 2.92 (d, *J =* 5.7 Hz, 2H), 2.59 (s, 5H). HRMS (ESI): calcd for C₂₁H₂₂NOs [M+H⁺]:368.1498, found 368.1491.

### 2-(2-Chloro benzylidene)-7-(3-hydroxy-1-methylpiperidin-4-yl)-4,6-dimethoxybenzo furan-3(2H)-one (136, Fig 3, Table-3):

TLC (DCM: MeOH 9:1): *R_{f}*= 0.5; Yellow solid; ¹H NMR (400 MHz, CDCl₃) δ 8.16 (d, *J* = 8.0 Hz, 1H), 7.41 (t, *J =* 7.7 Hz, 1H), 7.34 - 7.25 (m, 2H), 7.17 (s, 1H), 6.14 (s, 1H), 3.99 (s, 3H), 3.93 (s, 3H), 3.61 (m, 1H) 3.55 (d, *J=* 8.1 Hz, 2H), 3.18 (br s 1H), 2.82 - 2.63 (m, 7H), 1.82 (d*, J* = 12.3,2H).

### (Z)-2-(Furan-2-ylmethylene)-6-hydroxy-4-methoxy-7-(l-methylpiperidin-4-yl)benzo furan-3(2H)-one (137, Fig 3, Table-3):

TLC (DCM: MeOH 9:1): *R_{f}*= 0.5; Yellow solid; ¹H NMR (400 MHz, MeOD) δ 7.74 (s, 1H), 7.51 (s, 1H), 7.09 (s, 1H), 6.91 (s, 1H), 6.02 (s, 1H), 3.98 (s,3H) 3.57 (d, *J =* 11.2 Hz, 3H), 3.14 (d, *J* = 12.2 Hz, 2H), 2.85 (s, 3H), 2.59 (d, *J =* 12.5 Hz, 2H), 1.94 (d, *J* = 11.5 Hz, 2H).

### (E)-4-Ethoxy-2-(2-fluorobenzylidene)-7-(1-methyl-1,2,3,6-tetrahydropyridin-4-yl)benzofuran-3(2H)-one (138, Fig 3, Table-3):

TLC (DCM: MeOH 9:1): *R_{f}*= 0.5; Yellow solid; ¹H NMR (400 MHz, CDCl₃) δ 8.21 (t, *J =* 8.3 Hz, 1H), 7.47 (d, *J* = 8.6 Hz, 1H), 7.35 (dd, *J* = 13.5, 5.7 Hz, 1H), 7.22 (d, *J* = 7.4 Hz, 1H), 7.14 (d, *J =* 7.4 Hz, 1H), 7.10 (s, 1H), 6.59 (d*, J* = 8.6 Hz, 1H), 6.29 (s, 1H), 4.25 (q*, J =* 7.0 Hz, 2H), 3.27 (s, 2H), 2.79 (d, *J =* 5.2 Hz, 2H), 2.71 (s, 2H), 2.49 (s, 3H), 1.52 (t, *J =* 7.0 Hz, 3H).

### (Z)-2-(4-Bromobenzylidene)-4-ethoxy-7-(1-methyl-1,2,3,6-tetrahydropyridin-4-yl)benzo furan-3(2H)-one (I39, Fig3, Table-3):

TLC (DCM: MeOH 9:1): *R_{f}*= 0.5; Yellow solid; ¹H NMR (400 MHz, CDCl₃δ 7.74 (d*, J* = 8.5 Hz, 2H), 7.64 (d, *J* = 8.5 Hz, 2H), 7.52 (d, *J* = 8.5 Hz, 1H), 6.78 (s, 1H), 6.63 (d, *J* = 8.6 Hz, 1H) 6.29 (s, 1H), 4.25 (q, *J* = 7.0 Hz, 2H), 3.27 (s, 2H), 2.79 (d, *J* = 5.2 Hz, 2H), 2.71 (s, 2H), 2.49 (s, 3H), 1.52 (t*, J =* 7.0 Hz, 3H).

**Table 1**

| Entry | Code | Structure | Entry | Code | Structure |
|---|---|---|---|---|---|
| 1 | **I1** | 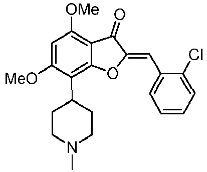 | 8 | **I8** | 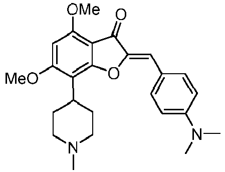 |
| 2 | **I2** | 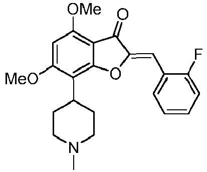 | 9 | **I9** | 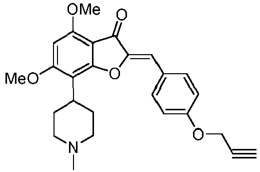 |
| 3 | **I3** | 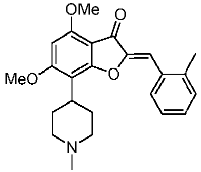 | 10 | **I10** | 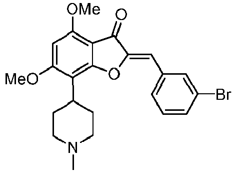 |
| 4 | **I4** | 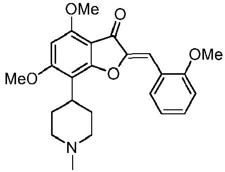 | 11 | **I11** | 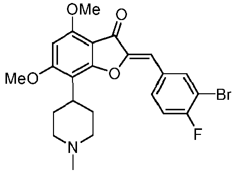 |
| 5 | **I5** | 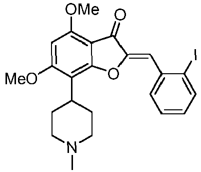 | 12 | **I12** | 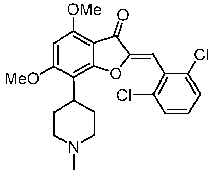 |
| 6 | **I6** | 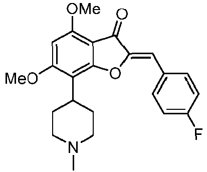 | 13 | **I13** | 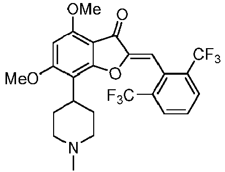 |
| 7 | **I7** | 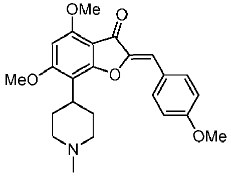 | 14 | **I14** | 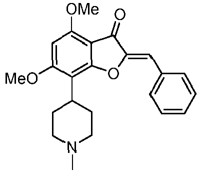 |

**Table 2**

| **Entry** | **Code** | **Structure** | **Entry** | **Code** | **Structure** |
|---|---|---|---|---|---|
| 1 | **I15** | 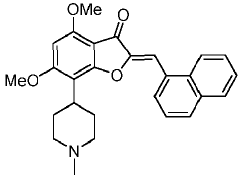 | 8 | **I22** | 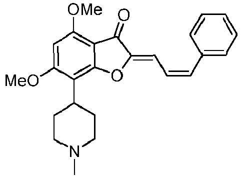 |
| 2 | **I16** | 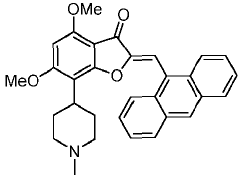 | 9 | **I23** | 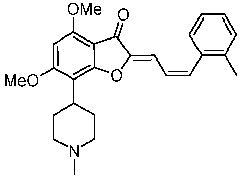 |
| 3 | I17 | 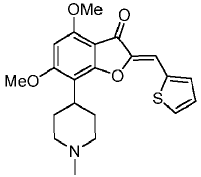 | 10 | **I24** | 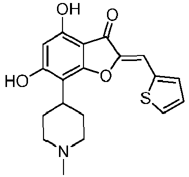 |
| 4 | I18 | 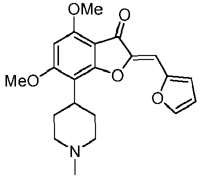 | 11 | **I25** | 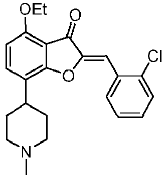 |
| 5 | I19 | 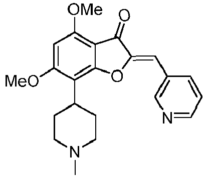 | **12** | **I26** | 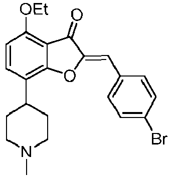 |
| 6 | **I20** | 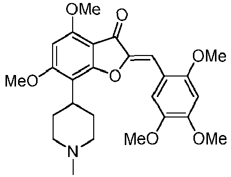 | 13 | **I27** | 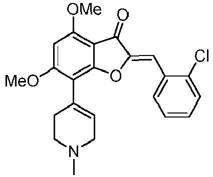 |
| 7 | **I21** | 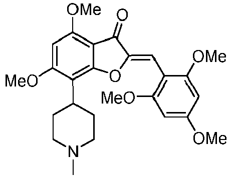 | 14 | **I28** | 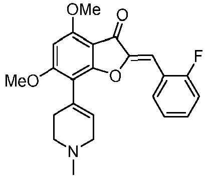 |

**Table 3**

| **Entry** | **Code** | **Structure** | **Entry** | **Code** | **Structure** |
|---|---|---|---|---|---|
| 1 | **I29** | 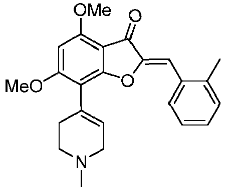 | 7 | **I35** | 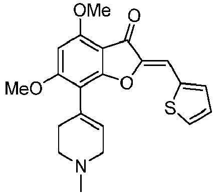 |
| 2 | **I30** | 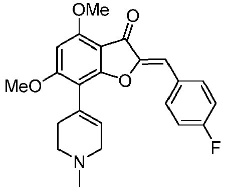 | 9 | **I36** | 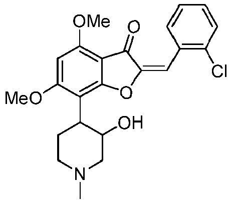 |
| 3 | **I31** | 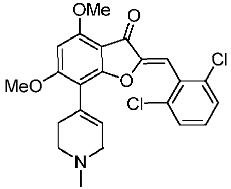 | 10 | **I37** | 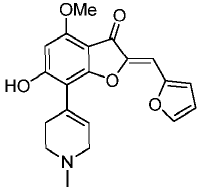 |
| 4 | **I32** | 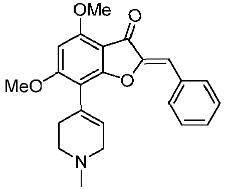 | 11 | **I38** | 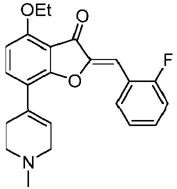 |
| 5 | **I33** | 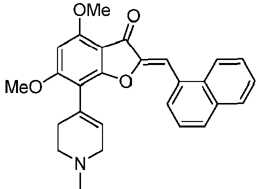 | 12 | **I39** | 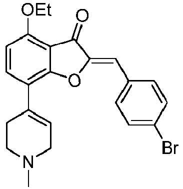 |
| 6 | **I34** | 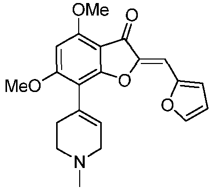 | | | |

### Biological Evaluation

### Example 1:

### In vitro activity of compounds I1 to 139 against M. tuberculosis H₃₇Rv

**MIC determination:** MIC was determined by broth dilution method against *M**.** tuberculosis* H₃₇Rv (ATCC 27294; American Type Culture Collection, Manassas, VA), *M**.** tuberculosis* MDR (resistant to isoniazid and rifampicin) and *M**.** tuberculosis* XDR (resistant to isoniazid , rifampicin, amikacin and moxifloxacin). The bacterial strains were grown for 10 to 15 days in Middlebrook 7H9 broth (Difco Laboratories, Detroit, Mich.) supplemented with 0.5% (v/v) glycerol, 0.25% (v/v) Tween 80 (Himedia, Mumbai India), and 10% ADC (albumin dextrose catalase, Becton Dickinson, Sparks, MD) under shaking conditions at 37 °C in 5% CO₂ to facilitate exponential-phase growth of the organism. Bacterial suspension was prepared by suspending *M**.** tuberculosis* growth in normal saline containing 0.5% tween 80 and turbidity was adjusted to 1 McFarland standard which is equivalent to 1.5 x 10⁷ CFU/ml. The 2-fold serial dilutions of compounds **I₁** to **I₃₉** were prepared in Middle brook 7H9 (Difco laboratories) for *M**.** tuberculosis* in 100 µl per well in 96-well U bottom microtitre plates (Tarson, Mumbai, India). The above-mentioned bacterial suspension was further diluted in the growth media and 100 µl volume of this diluted inoculum was added to each well of the plate resulting in the final inoculum of 5 x 10⁵ CFU/ml in the well and the final concentrations of compound **I₁** to I**₃₉** ranged from 0.5 to 64 µg/ml (0.5, 1, 2, 4, 8, 16, 32 64). The plates were incubated at 37 °C for 3-weeks in 5% CO₂. The plates were read visually and the minimum concentration of the compound showing no turbidity was recorded as MIC.

### Results:

The compounds **I1** to **I39** were screened against H₃₇Rv of *M**.** Tuberculosis* and the results were summarised in Table 4. All the compounds have shown MIC in the range of 1-64 µg/ml. Among all, ten compounds **(I1, I2, I3, I7, I10, I15, I16, I25, I27** and **I38)** have shown potent MIC in the range of 1-8 µg/ml.

**Table 4**

| **S.No.** | **Compoun d code** | **MIC (µg/ml)** | **S.No.** | **Compound code** | **MIC (µg/ml)** |
|---|---|---|---|---|---|
| 1. | **I1** | 4 | 21. | **I21** | 32 |
| 2. | **I2** | 4 | 22. | **I22** | 32 |
| 3. | **I3** | 8 | 23. | **I23** | 16 |
| 4. | **I4** | >64 | 24. | **I24** | 64 |
| 5. | **I5** | >64 | 25. | **I25** | 4 |
| 6. | **I6** | 8 | 26. | **I26** | 32 |
| 7. | **I7** | 32 | 27. | **I27** | 1 |
| 8. | **I8** | 16 | 28. | **I28** | 16 |
| 9. | **I9** | 8 | 29. | **I29** | 64 |
| 10. | **I10** | 16 | 30. | **I30** | 16 |
| 11. | **I11** | 16 | 31. | **I31** | 32 |
| 12. | **I12** | >64 | 32. | **I32** | 64 |
| 13. | **I13** | 32 | 33. | **I33** | 64 |
| 14. | **I14** | >64 | 34. | **I34** | 32 |
| 15. | **I15** | 8 | 35. | **I35** | 16 |
| 16. | **I16** | 2 | 36. | **I36** | 16 |
| 17. | **I17** | 32 | 37. | **I37** | 64 |
| 18. | **I18** | 16 | 38. | **I38** | 8 |
| 19. | **I19** | >64 | 39. | **I39** | 32 |
| 20. | **I20** | 64 | 40. | **Rifampicin** | 0.06 |

## Claims

1. A compound of formula I or pharmaceutically acceptable salts thereof, 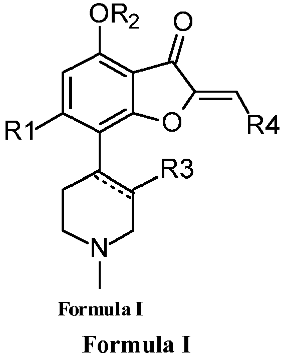 wherein
R₁ is selected from the group consisting of H, OH, alkoxy,
R₂ is selected from the group consisting of CF₃, alkyl,
---- Represents an optional second carbon - carbon bond,
R₃ is selected from the group consisting of H, OH,
R₄ is selected from the group consisting of alkyl, substituted alkyl, styryl, substituted styryl, aryl, substituted aryl, biaryl, substituted biaryl, napthyl, anthracenyl, substituted or unsubstituted heterocycles or heteroaryl and heterocycle or heteroaryl is selected from the group consisting of furanyl, thiophenyl, perazinyl, morpholinyl, piperidyl, pyridyl, triazolyl, triazinyl, pyrimidinyl, pyridazinyl, oxazolyl, benzofuranyl, pyrrolyl, imidazoyl, thiazoyl, quinolinyl, isoquinolinyl, benzooxazolyl, benzothiazolyl and further substituted aryl, substituted styryl, substituted vinyl, substituted biaryl and substituted heteroaryl or heterocycle wherein the substitution ranges from 1 to 4 and the substituents are independently selected from the group consisting of F, Cl, I, NR_{I1}R_{I2}, CF₃, OCF₃, OR_{I3}, NO₂, alkyl chain from C₁ to C₁₄,;
R_{I1}, R_{I2} and R_{I3} are each independently selected from the group consisting of H, alkyl, substituted alkyl phenyl, substituted phenyl, propargyl.

2. The compound as claimed in claim 1, wherein representative compounds are:
(*E*)-2-(2-Chlorobenzylidene)-4,6-dimethoxy-7-(1-methylpiperidin-4-yl)benzofuran-3(2*H*)-one(Compound **I1**)
(*E*)-2-(2-Fluorobenzylidene)-4,6-dimethoxy-7-(1-methylpiperidin-4-yl)benzofuran-3(2*H*)-one (Compound **12)**
(*Z*)-4,6-Dimethoxy-2-(2-methylbenzylidene)-7-(1-methylpiperidin-4-yl)benzofuran-3(2*H*)-one (Compound **I3)**
(*E*)-4,6-Dimethoxy-2-(2-methoxybenzylidene)-7-(1-methylpiperidin-4-yl)benzofuran-3(2*H*)-one (Compound **I4)**
(*E*)-2-(2-Iodobenzylidene)-4,6-dimethoxy-7-(1-methylpiperidin-4-yl)benzofuran-3(2*H*)-one (Compound**15**) (*Z*)-2-(4-Fluorobenzylidene)-4,6-dimethoxy-7-(1-methylpiperidin-4-yl)benzofuran-3(2*H*)-one (Compound **I6)**
(*Z*)-4,6-Dimethoxy-2-(4-methoxybenzylidene)-7-(1-methylpiperidin-4-yl)benzofuran-3(2H)-one (Compound **I7)**
(*Z*)-2-[4-(Dimethylamino)benzylidene]-4,6-dimethoxy-7-(1-methylpiperidin-4-yl)benzofuran-3(2*H*)-one (Compound **I8)**
(Z)-4,6-Dimethoxy-7-(1-methylpiperidin-4-yl)-2-[4-(prop-2-yn-1-yloxy)benzylidene]benzofuran-3(2*H*)-one (Compound **I9)**
(*Z*)-2-(2,6-Dichlorobenzylidene)-4,6-dimethoxy-7-(1-methylpiperidin-4-yl)benzofuran3(2*H*)-one (Compound **I12**)
(*Z*)-2-[2,6-Bis(trifluoromethyl)benzylidene]-4,6-dimethoxy-7-(1-methylpiperidin-4yl)benzofuran-3(2*H*)-one(Compound **I13)**
(*Z*)-2-Benzylidene-4,6-dimethoxy-7-(1-methylpiperidin-4-yl)benzofuran-3(2*H*)-one (Compound **I14,)** (*E*)-4,6-Dimethoxy-7-(1-methylpiperidin-4-yl)-2-(naphthalen-1-ylmethylene)benzofuran -3(2*H*)-one (Compound **I15)**
(*E*)-2-(Anthracen-9-ylmethylene)-4,6-dimethoxy-7-(1-methylpiperidin-4-yl)benzofuran-3(2*H*)-one (Compound **I16)**
(*E*)-4,6-dimethoxy-7-(1-methylpiperidin-4-yl)-2-(thiophen-2-ylmethylene)benzofuran-3(2*H*)-one (Compound **I17)**
(Z)-2-(Furan-2-ylmethylene)-4,6-dimethoxy-7-(1-methylpiperidin-4-yl)benzofuran-3(2*H*)-one (Compound **I18)**
(*E*)-4,6-Dimethoxy-7-(1-methylpiperidin-4-yl)-2-(pyridin-3-ylmethylene)benzofuran-3(2*H*)-one (Compound **I19,** Table 2)
(*Z*)-4,6-Dimethoxy-7-(1-methylpiperidin-4-yl)-2-(2,4,5-trimethoxybenzylidene)benzofuran-3(2*H*)-one (Compound **I20)**
(*Z*)-4,6-Dimcthoxy-7-(1-methylpiperidin-4-yl)-2-(2,4,6-trimethoxybenzylidene)benzofuran-3(2*H*)-one (Compound **I21)**
(*E*)-4,6-Dimethoxy-7-(1-methylpiperidin-4-yl)-2-((*Z*)-3-phenylallylidene)benzofuran-3(2*H*)-one (Compound **I22)**
(*Z*)-4,6-Dimethoxy-7-(1-methylpiperidin-4-yl)-2-((Z)-3-(o-tolyl)allylidene)benzofuran-3(2*H*)-one (Compound **I23,** Table 2)
(*E*)-2-(2-Chlorobenzylidene)-4-ethoxy-7-(1-methylpiperidin-4-yl)benzofuran-3(2*H*)-one (Compound **I25)** (*E*)-2-(2-Chlorobenzylidene)-4,6-dimethoxy-7-(1-methyl-1,2,3,6-tetrahydropyridin-4-yl)benzofuran-3(2*H*)-one (Compound **I27)**
(*E*)-2-(2-Fluorobenzylidene)-4,6-dimethoxy-7-(1-methyl-1,2,3,6-tetrahydropyridin-4-yl) benzofuran-3(2*H*)-one (Compound **I28)**
(*E*)-4,6-Dimethoxy-7-(1-methyl-1,2,3,6-tetrahydropyridin-4-yl)-2-(2-methylbenzylide-ne)benzofuran-3(2*H*)-one (Compound **I29,** Table 3)
(*Z*)-2-(4-Fluorobenzylidene)-4,6-dimethoxy-7-(1-methyl-1,2,3,6-tetrahydropyridin-4-yl)benzofuran-3(2*H*)-one (Compound **I30)**
(*Z*)-2-(2,6-Dichlorobenzylidene)-4,6-dimethoxy-7-(1-methyl-1,2,3,6-tetrahydropyridin-4-yl)benzofuran-3(2*H*)-one (Compound **I31)**
(*Z*)-2-Benzylidene-4,6-dimethoxy-7-(1-methyl-1,2,3,6-tetrahydropyridin-4-yl)benzofuran-3(2*H*)-one (Compound **I32)**
(*E*)-4,6-Dimethoxy-7-(1-methyl-1,2,3,6-tetrahydropyridin-4-yl)-2-(naphthalen-1-ylme-thylene)benzofuran-3(2*H*)-one (Compound **I33)**
(*Z*)-2-(Furan-2-ylmethylene)-4,6-dimethoxy-7-(1-methyl-1,2,3,6-tetrahydropyridin-4-yl)benzofuran-3(2*H*)-one (Compound **I34)**
(*E*)-4,6-Dimethoxy-7-(1-methyl-1,2,3,6-tetrahydropyridin-4-yl)-2-(thiophen-2-ylmethylene)benzofuran-3(2*H*)-one (Compound **I35)**
(*E*)-2-(2-Chlorobenzylidene)-7-(3-hydroxy-1-methylpiperidin-4-yl)-4,6-dimethoxy benzofuran-3(2*H*)-one (Compound **I36),**
(*Z*)-2-(Furan-2-ylmethylene)-4-hydroxy-6-methoxy-7-(1-methylpiperidin-4-yl)benzo furan-3(2*H*)-one (Compound **I37**)(*E*)-4-Ethoxy-2-(2-tluorobenzylidene)-7-(1-methyl-1,2,3,6-tetrahydropyridin-4-yl)ben zofuran-3(2H)-one (Compound **I38).**

3. A compound
(*Z*)-2-(3-Bromobenzylidene)-4,6-dimethoxy-7-(1-methylpiperidin-4-yl)benzofuran-3(2*H*)-one(Compound I10);
(*Z*)-2-(3-Bromo-4-fluorobenzylidene)-4,6-dimethoxy-7-(1-methylpiperidin-4-yl)benzofuran-3(2*H*)-one (Compound I11);
(*E*)-4,6-Dihydroxy-7-(1-methylpiperidin-4-yl)-2-(thiophen-2-ylmethylene)benzofuran-3(2H)-one (Compound **I24)**
(Z)-2-(4-Bromobenzylidene)-4-ethoxy-7-(1-methylpiperidin-4-yl)benzofuran-3(2*H*)-one (Compound 126); and
(Z)-2-(4-Bromobenzylidene)-4-ethoxy-7-(1-methyl-1,2,3,6-tetrahydropyridin-4-yl)benzo-furan-3(2*H*)-one (Compound 139) or pharmaceutically acceptable salts thereof.

4. The compound as claimed in claim 1 or claim 3, for use in treatment of tuberculosis.

5. The compound as claimed in claim 1 or claim 3, wherein the pharmaceutically acceptable salts are salts of an acid selected from the group consisting of hydrochloric acid, sulphuric acid, phosphoric acid, acetic acid, citric acid, oxalic acid, malonic acid, salyilic acid, malic acid, fumaric acid, succinic acid, ascorbic acid, maleic acid, methanesulfonic acid, and isoethonic acids.

6. A process for preparation of compounds as claimed in claim 1 and claim 3 wherein the process steps comprising;
(i) reacting compound of formula 2 or 3 with an aldehyde (formula 4) compound in presence of base in protic solvent at a temperature ranging between 20 to 100°C for a period in the range of 2 to 12 hr to obtain compound of formula 6 or 5 respectively, 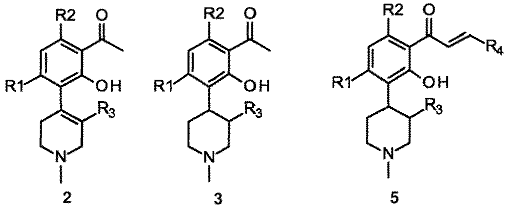 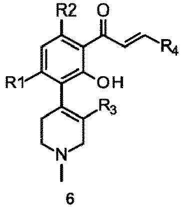
(ii) reacting compound of formula 5 or 6 with Hg(OAc)₂ in presence of base at a temperature in the range of 60 to 150 °C for a period in the range of 1 to 12 hr to obtain compound of formula I or any of compounds 110. I11, I24, I26 or 139 as defined in claim 3.

7. The process as claimed in claim 6, wherein the aldehyde is selected from a group consisting of alkyl, substituted alkyl, styryl, substituted styryl, aryl, substituted aryl, biaryl, substituted biaryl, napthyl, anthracenyl, substituted or unsubstituted heterocycles or heteroaryl and heterocycle or heteroaryl is selected from the group consisting of furanyl, thiophenyl, perazinyl, morpholinyl, piperidyl, pyridyl, triazolyl, triazinyl, pyrimidinyl, pyridazinyl, oxazolyl, benzofuranyl, pyrrolyl, imidazoyl, thiazoyl, quinolinyl, isoquinolinyl, benzooxazolyl, benzothiazolyl and further substituted aryl, substituted styryl, substituted vinyl, substituted biaryl and substituted heterocycle or heteroaryl wherein the substitution ranges from 1 to 4 and the substituents are independently selected from the group consisting of F, Cl, Br, I, NR_{I1}R_{I2}, CF₃, OCF₃, OR_{I3}, NO₂, alkyl chain from C₁ to C₁₄,
R_{I1}, R_{I2} and R_{I3} are each independently selected from the group consisting of H, alkyl, substituted alkyl phenyl, substituted phenyl.

8. The process as claimed in claim 6 wherein the base used in step i) is selected from a group consisting of KOH, NaOH, K₂CO₃, Cs₂CO₃.

9. The process as claimed in claim 6 wherein the base used in step ii) is selected from a group consisting pyridine or triethylamine.

10. The process as claimed in claim 6, wherein the protic solvent used is selected from a group consisting of methanol, ethanol and water.

11. A pharmaceutical composition comprising the compound of formula 1 as defined in claim 1 or compounds I10 or I11 or 124 or 126 or 139 as defined in claim 3, optionally along with a pharmaceutically acceptable carrier, salt, excipients or diluents.

## Patentansprüche

1. Verbindung der Formel I oder pharmazeutisch annehmbare Salze hiervon, 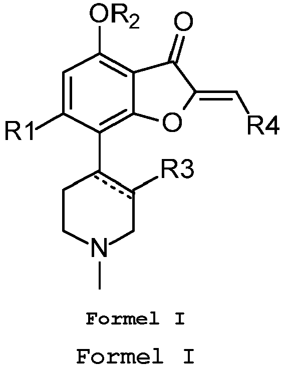 wobei
R₁ ausgewählt ist aus der Gruppe bestehend aus H, OH, Alkoxy,
R₂ ausgewählt ist aus der Gruppe bestehend aus CF₃, Alkyl,
--- eine optionale zweite Kohlenstoff-Kohlenstoff-Bindung darstellt,
R₃ ausgewählt ist aus der Gruppe bestehend aus H, OH,
R₄ ausgewählt ist aus der Gruppe bestehend aus Alkyl, substituiertem Alkyl, Styryl, substituiertem Styryl, Aryl, substituiertem Aryl, Biaryl, substituiertem Biaryl, Naphthyl, Anthracenyl, substituierten oder unsubstituierten Heterocyclen oder Heteroaryl und Heterocyclus oder Heteroaryl ausgewählt ist aus der Gruppe bestehend aus Furanyl, Thiophenyl, Perazinyl, Morpholinyl, Piperidyl, Pyridyl, Triazolyl, Triazinyl, Pyrimidinyl, Pyridazinyl, Oxazolyl, Benzofuranyl, Pyrrolyl, Imidazolyl, Thiazolyl, Chinolinyl, Isochinolinyl, Benzoxazolyl, Benzothiazolyl und weiter substituiertem Aryl, substituiertem Styryl, substituiertem Vinyl, substituiertem Biaryl und substituiertem Heteroaryl oder Heterocyclus, wobei die Substitution im Bereich von 1 bis 4 liegt und die Substituenten unabhängig voneinander ausgewählt sind aus der Gruppe bestehend aus F, Cl, I, NR_{I1}R_{I2}, CF₃, OCF₃, OR_{I3}, NO₂, Alkylkette von C₁ bis C₁₄;
R_{I1}, R_{I2} und R_{I3} jeweils unabhängig voneinander ausgewählt sind aus der Gruppe bestehend aus H, Alkyl, substituiertem Alkylphenyl, substituiertem Phenyl, Propargyl.

2. Verbindung nach Anspruch 1, wobei repräsentative Verbindungen sind:
(*E*)-2-(2-Chlorobenzyliden)-4,6-dimethoxy-7-(1-methylpiperidin-4-yl)benzofuran-3(2*H*)-on (Verbindung **I1)**
(*E*)-2-(2-Fluorobenzyliden)-4,6-dimethoxy-7-(1-methylpiperidin-4-yl)benzofuran-3(2*H*)-on (Verbindung **I2)**
(*Z*)-4,6-Dimethoxy-2-(2-methylbenzyliden)-7-(1-methylpiperidin-4-yl)benzofuran-3(2*H*)-on (Verbindung **I3)**
(*E*)-4,6-Dimethoxy-2-(2-methoxybenzyliden)-7-(1-methylpiperidin-4-yl)benzofuran-3(2*H*)-on (Verbindung **I4)**
(*E*)-2-(2-Iodobenzyliden)-4,6-dimethoxy-7-(1-methylpiperidin-4-yl)benzofuran-3(2*H*)-on (Verbindung **I5)**
(*Z*)-2-(4-Fluorobenzyliden)-4,6-dimethoxy-7-(1-methylpiperidin-4-yl)benzofuran-3(2*H*)-on (Verbindung **I6)**
(*Z*)-4,6-Dimethoxy-2-(4-methoxybenzyliden)-7-(1-methylpiperidin-4-yl)benzofuran-3(2*H*)-on (Verbindung **I7)**
(*Z*)-2-[4-(Dimethylamino)benzyliden]-4,6-dimethoxy-7-(1-methylpiperidin-4-yl)benzofuran-3(2*H*)-on (Verbindung **I8)**
(*Z*)-4,6-Dimethoxy-7-(1-methylpiperidin-4-yl)-2-[4-(prop-2-yn-1-yloxy)benzyliden] benzofuran-3(2*H*)-on (Verbindung **I9)**
(*Z*)-2-(2,6-Dichlorobenzyliden)-4,6-dimethoxy-7-(1-methylpiperidin-4-yl)benzofuran-3(2*H*)-on (Verbindung **I12)**
(*Z*)-2-[2,6-Bis(trifluoromethyl)benzyliden]-4,6-dimethoxy-7-(1-methylpiperidin-4yl)benzofuran-3(2*H*)-on (Verbindung **I13)**
(*Z*)-2-Benzyliden-4,6-dimethoxy-7-(1-methylpiperidin-4-yl)benzofuran-3(2*H*)-on (Verbindung **I14)**
(*E*)-4,6-Dimethoxy-7-(1-methylpiperidin-4-y1)-2-(naphthalen-1-ylmethylen)benzofuran-3(2*H*)-on (Verbindung **I15)**
(*E*)-2-(Anthracen-9-ylmcthylen)-4,6-dimethoxy-7-(1-methylpiperidin-4-yl)benzofuran-3(2*H*)-on (Verbindung **I16)**
(*E*)-4,6-dimethoxy-7-(1-methylpiperidin-4-yl)-2-(thiophen-2-ylmethylen)benzofuran-3(2*H*)-on (Verbindung **I17)**
(*Z*)-2-(Furan-2-ylmethylen)-4,6-dimethoxy-7-(1-methylpiperidin-4-yl)benzofuran-3(2*H*)-on (Verbindung **I18)**
(*E*)-4,6-Dimethoxy-7-(1-methylpiperidin-4-yl)-2-(pyridin-3-ylmethylen)benzofuran-3(2*H*)-on (Verbindung **I19,** Tabelle 2)
(*Z*)-4,6-Dimethoxy-7-(1-methylpiperidin-4-yl)-2-(2,4,5-trimethoxybenzyliden)benzofuran-3(2*H*)-on (Verbindung **I20)**
(*Z*)-4,6-Dimethoxy-7-(1-methylpiperidin-4-yl)-2-(2,4,6-trimethoxybenzyliden)benzofuran-3(2H)-on (Verbindung **I21)**
(*E*)-4,6-Dimethoxy-7-(1-methylpiperidin-4-y1)-2-((Z)-3-phenylallyliden)benzofuran-3(2*H*)-on (Verbindung **I22)**
(*Z*)-4,6-Dimethoxy-7-(1-methylpiperidin-4-yl)-2-((Z)-3-(o-tolyl)allyliden)benzofuran-3(2*H*)-on (Verbindung **I23,** Tabelle 2)
(E)-2-(2-Chlorobenzyliden)-4-ethoxy-7-(1-methylpiperidin-4-yl)benzofuran-3(2*H*)-on (Verbindung **I25)**
(*E*)-2-(2-Chlorobenzyliden)-4,6-dimethoxy-7-(1-methyl-1,2,3,6-tetrahydropyridin-4-yl)benzofuran-3(2*H*)-on (Verbindung **I27)**
(*E*)-2-(2-Fluorobenzyliden)-4,6-dimethoxy-7-(1-methyl-1,2,3,6-tetrahydropyridin-4-yl)benzofuran-3(2*H*)-on (Verbindung **I28)**
(*E*)-4,6-Dimethoxy-7-(1-methyl-1,2,3,6-tetrahydropyridin-4-yl)-2-(2-methylbenzyliden)benzofuran-3(2H)-on (Verbindung **I29,** Tabelle 3)
(*Z*)-2-(4-Fluorobenzyliden)-4,6-dimethoxy-7-(1-methyl-1,2,3,6-tetrahydropyridin-4-yl)benzofuran-3(2*H*)-on (Verbindung **I30)**
(Z)-2-(2,6-Dichlorobenzyliden)-4,6-dimethoxy-7-(1-methyl-1,2,3,6-tetrahydropyridin-4-yl)benzofuran-3(2H)-on (Verbindung **I31)**
(*Z*)-2-Benzyliden-4,6-dimethoxy-7-(1-methyl-1,2,3,6-tetrahydropyridin-4-yl)benzofuran-3(2*H*)-on (Verbindung **I32)**
(*E*)-4,6-Dimethoxy-7-(1-methyl-1,2,3,6-tetrahydropyridin-4-yl)-2-(naphthalen-1-ylmethylen)benzofuran-3(2*H*)-on (Verbindung **I33)**
(*Z*)-2-(Furan-2-ylmethylen)-4,6-dimethoxy-7-(1-methyl-1,2,3,6-tetrahydropyridin-4-yl)benzofuran-3(2H)-on (Verbindung **I34)**
(*E*)-4,6-Dimethoxy-7-(1-methyl-1,2,3,6-tetrahydropyridin-4-yl)-2-(thiophen-2-ylmethylen)benzofuran-3(2*H*)-on (Verbindung **I35)**
(*E*)-2-(2-Chlorobenzyliden)-7-(3-hydroxy-1-methylpiperidin-4-yl)-4,6-dimethoxy benzofuran-3(2*H*)-on (Verbindung **I36),**
(*Z*)-2-(Furan-2-ylmethylen)-4-hydroxy-6-methoxy-7-(1-methylpiperidin-4-yl)benzofuran-3(2*H*)-on (Verbindung **I37)**
(*E*)-4-Ethoxy-2-(2-fluorobenzyliden)-7-(1-methyl-1,2,3,6-tetrahydropyridin-4-yl)benzofuran-3(2*H*)-on (Verbindung **I38).**

3. Verbindung
(*Z*)-2-(3-Bromobenzyliden)-4,6-dimethoxy-7-(1-methylpiperidin-4-yl)benzofuran-3(2H)-on (Verbindung **I10);**
(*Z*)-2-(3-Bromo-4-fluorobenzyliden)-4,6-dimethoxy-7-(1-methylpiperidin-4-yl)benzofuran-3(2H)-on (Verbindung **I11);**
(*E*)-4,6-Dihydroxy-7-(1-methylpiperidin-4-yl)-2-(thiophen-2-ylmethylen)benzofuran-3(2*H*)-on (Verbindung **I24)**
(*Z*)-2-(4-Bromobenzyliden)-4-ethoxy-7-(1-methylpiperidin-4-yl)benzofuran-3(2H)-on (Verbindung **I26);** und
(*Z*)-2-(4-Bromobenzyliden)-4-ethoxy-7-(1-methyl-1,2,3,6-tetrahydropyridin-4-yl)benzofuran-3(2*H*)-on (Verbindung **I39)**
oder pharmazeutisch annehmbare Salze hiervon.

4. Verbindung nach Anspruch 1 oder Anspruch 3 zur Verwendung bei der Behandlung von Tuberkulose.

5. Verbindung nach Anspruch 1 oder Anspruch 3, wobei die pharmazeutisch annehmbaren Salze Salze einer Säure sind, ausgewählt aus der Gruppe bestehend aus Salzsäure, Schwefelsäure, Phosphorsäure, Essigsäure, Zitronensäure, Oxalsäure, Malonsäure, Salyilsäure, Apfelsäure, Fumarsäure, Bernsteinsäure, Ascorbinsäure, Maleinsäure, Methansulfonsäure und Isoethonsäuren.

6. Verfahren zur Herstellung von Verbindungen nach Anspruch 1 und Anspruch 3, wobei das Verfahren die folgenden Schritte umfasst:
(i) Umsetzen der Verbindung der Formel 2 oder 3 mit einer Aldehydverbindung (Formel 4) in Gegenwart einer Base in protischem Lösungsmittel bei einer Temperatur im Bereich von 20 bis 100°C für einen Zeitraum im Bereich von 2 bis 12 Stunden, um die Verbindung der Formel 6 bzw. 5 zu erhalten, 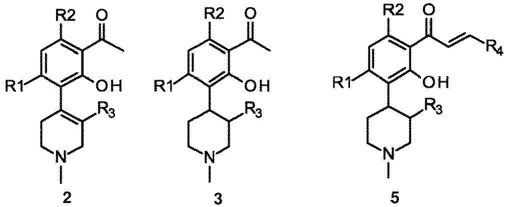 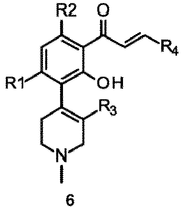
(ii) Umsetzen der Verbindung der Formel 5 oder 6 mit Hg(OAc)₂ in Gegenwart einer Base bei einer Temperatur im Bereich von 60 bis 150°C für einen Zeitraum im Bereich von 1 bis 12 Stunden, um die Verbindung der Formel I oder eine der Verbindungen I10, I11, I24, I26 oder I39 nach Anspruch 3 zu erhalten.

7. Verfahren nach Anspruch 6, wobei das Aldehyd ausgewählt ist aus einer Gruppe bestehend aus Alkyl, substituiertem Alkyl, Styryl, substituiertem Styryl, Aryl, substituiertem Aryl, Biaryl, substituiertem Biaryl, Naphthyl, Anthracenyl, substituierten oder unsubstituierten Heterocyclen oder Heteroaryl, und Heterocyclus oder Heteroaryl ausgewählt ist aus der Gruppe bestehend aus Furanyl, Thiophenyl, Perazinyl, Morpholinyl, Piperidyl, Pyridyl, Triazolyl, Triazinyl, Pyrimidinyl, Pyridazinyl, Oxazolyl, Benzofuranyl, Pyrrolyl, Imidazolyl, Thiazolyl, Chinolinyl, Isochinolinyl, Benzoxazolyl, Benzothiazolyl und weiter substituiertem Aryl, substituiertem Styryl, substituiertem Vinyl, substituiertem Biaryl und substituiertem Heterocyclus oder Heteroaryl, wobei die Substitution im Bereich von 1 bis 4 liegt und die Substituenten unabhängig ausgewählt sind aus der Gruppe bestehend aus F, Cl, Br, I, NR_{I1}R_{I2}, CF₃, OCF₃, OR_{I3}, NO₂, Alkylkette von C₁ bis C₁₄,
R_{I1}, R_{I2} und R_{I3} jeweils unabhängig voneinander ausgewählt sind aus der Gruppe bestehend aus H, Alkyl, substituiertem Alkylphenyl, substituiertem Phenyl.

8. Verfahren nach Anspruch 6, wobei die in Schritt i) verwendete Base ausgewählt ist aus einer Gruppe bestehend aus KOH, NaOH, K₂CO₃, Cs₂CO₃.

9. Verfahren nach Anspruch 6, wobei die in Schritt ii) verwendete Base ausgewählt ist aus einer Gruppe bestehend aus Pyridin oder Triethylamin.

10. Verfahren nach Anspruch 6, wobei das verwendete protische Lösungsmittel ausgewählt ist aus einer Gruppe bestehend aus Methanol, Ethanol und Wasser.

11. Pharmazeutische Zusammensetzung, die die Verbindung der Formel 1 nach Anspruch 1 oder die Verbindungen I10 oder I11 oder I24 oder I26 oder I39 nach Anspruch 3 umfasst, gegebenenfalls zusammen mit einem pharmazeutisch annehmbaren Träger, Salz, Hilfsstoffen oder Verdünnern.

## Revendications

1. Composé de formule I ou sels pharmaceutiquement acceptables de celui-ci, 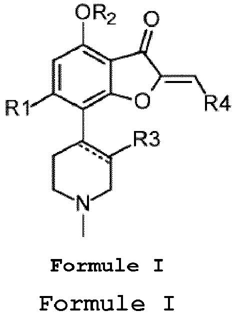 dans laquelle
R₁ est choisi dans le groupe constitué de H, OH, alcoxy,
R₂ est choisi dans le groupe constitué de CF₃, alkyle,
---- représente une deuxième liaison carbone - carbone éventuelle,
R₃ est choisi dans le groupe constitué de H, OH,
R₄ est choisi dans le groupe constitué de alkyle, alkyle substitué, styryle, styryle substitué, aryle, aryle substitué, biaryle, biaryle substitué, naphtyle, anthracényle, hétérocycles ou hétéroaryle substitués ou non substitués et hétérocycle ou hétéroaryle est choisi dans le groupe constitué de furanyle, thiophényle, pérazinyle, morpholinyle, pipéridyle, pyridyle, triazolyle, triazinyle, pyrimidinyle, pyridazinyle, oxazolyle, benzofuranyle, pyrrolyle, imidazolyle, thiazoyle, quinolinyle, isoquinolinyle, benzooxazolyle, benzothiazolyle et en outre aryle substitué, styryle substitué, vinyle substitué, biaryle substitué et hétéroaryle ou hétérocycle substitué, dans lesquels la substitution va de 1 à 4 et les substituants sont choisis indépendamment dans le groupe constitué de F, Cl, I, NR_{I1}R_{I2}, CF₃, OCF₃, OR₁₃, NO₂, chaîne alkyle de C₁ à C₁₄ ;
R_{I1}, R_{I2}, et R_{I3} sont chacun choisis indépendamment dans le groupe constitué de H, alkyle, alkylphényle substitué, phényle substitué, propargyle.

2. Composé selon la revendication 1, dans lequel les composés représentatifs sont :
(*E*)-2-(2-chlorobenzylidène)-4,6-diméthoxy-7-(1-méthylpipéridin-4-yl)benzofuran-3(2H)-one (composé **I1)**
(*E*)-2-(2-fluorobenzylidène)-4,6-diméthoxy-7-(1-méthylpipéridin-4-yl)benzofuran-3(2H)-one (composé **I2)**
(*Z*)-4,6-diméthoxy-2-(2-méthylbenzylidène)-7-(1-méthylpipéridin-4-yl)benzofuran-3(2H)-one (composé **I3)**
(*E*)-4,6-diméthoxy-2-(2-méthoxybenzylidène)-7-(1-méthylpipéridin-4-yl)benzofuran-3(2H)-one (composé **I4)**
(*E*)-2-(2-iodobenzylidène)-4,6-diméthoxy-7-(1-méthylpipéridin-4-yl)benzofuran-3(2H)-one (composé **I5)**
(*Z*)-2-(4-fluorobenzylidène)-4,6-diméthoxy-7-(1-méthylpipéridin-4-yl)benzofuran-3(2H)-one (composé **I6)**
(*Z*)-4,6-diméthoxy-2-(4-méthoxybenzylidène)-7-(1-méthylpipéridin-4-yl)benzofuran-3(2H)-one (composé **I7)**
(Z)-2-[4-(diméthylamino)benzylidène]-4,6-diméthoxy-7-(1-méthylpipéridin-4-yl)benzofuran-3(2H)-one (composé **I8)**
(Z)-4,6-diméthoxy-7-(1-méthylpipéridin-4-yl)-2-[4-(prop-2-yn-1-yloxy)benzylidène]benzofuran-3(2*H*)-one (composé **I9)**
(*Z*)-2-(2,6-dichlorobenzylidène)-4,6-diméthoxy-7-(1-méthylpipéridin-4-yl)benzofuran-3(2H)-one (composé **I12)**
(*Z*)-2-[2,6-bis(trifluorométhyl)benzylidène]-4,6-diméthoxy-7-(1-méthylpipéridin-4-yl)benzofuran-3(2*H*)-one (composé **I13)**
(*Z*)-2-benzylidène-4,6-diméthoxy-7-(1-méthylpipéridin-4-yl)benzofuran-3(2H)-one (composé **I14)**
(*E*)-4,6-diméthoxy-7-(1-méthylpipéridin-4-yl)-2-(naphtalèn-1-ylméthylène)benzofuran-3(2*H*)-one (composé **I15)**
(*E*)-2-(anthracèn-9-ylméthylène)-4,6-diméthoxy-7-(1-méthylpipéridin-4-yl)-benzofuran-3(2H)-one (composé **I16)**
(*E*)-4,6-diméthoxy-7-(1-méthylpipéridin-4-yl)-2-(thiophèn-2-ylméthylène)benzofuran-3(2H)-one (composé **I17)**
(*Z*)-2-(furan-2-ylméthylène)-4,6-diméthoxy-7-(1-méthylpipéridin-4-yl)-benzofuran-3(2H)-one (composé **I18)**
(*E*)-4,6-diméthoxy-7-(1-méthylpipéridin-4-yl)-2-(pyridin-3-ylméthylène)benzofuran-3(2H)-one (composé **I19,** tableau 2)
(*Z*)-4,6-diméthoxy-7-(1-méthylpipéridin-4-yl)-2-(2,4,5-triméthoxybenzylidène)benzofuran-3(2H)-one (composé **I20)**
(*Z*)-4,6-diméthoxy-7-(1-méthylpipéridin-4-yl)-2-(2,4,6-triméthoxybenzylidène)benzofuran-3(2H)-one (composé **I21)**
(*E*)-4,6-diméthoxy-7-(1-méthylpipéridin-4-yl)-2-((Z)-3-phénylallylidène)benzofuran-3(2H)-one (composé **I22)**
(Z)-4,6-diméthoxy-7-(1-méthylpipéridin-4-yl)-2-((Z)-3-(o-tolyl)allylidène)benzofuran-3(2H)-one (composé **I23,** tableau 2)
(*E*)-2-(2-chlorobenzylidène)-4-éthoxy-7-(1-méthylpipéridin-4-yl)benzofuran-3(2H)-one (composé **I25)**
(*E*)-2-(2-chlorobenzylidène)-4,6-diméthoxy-7-(1-méthyl-1,2,3,6-tétrahydropyridin-4-yl)benzofuran-3(2H)-one (composé **I27)**
(E)-2-(2-fluorobenzylidène)-4,6-diméthoxy-7-(1-méthyl-1,2,3,6-tétrahydropyridin-4-yl)benzofuran-3(2H)-one (composé **I28)**
(*E*)-4,6-diméthoxy-7-(1-méthyl-1,2,3,6-tétrahydropyridin-4-yl)-2-(2-méthylbenzylidène)benzofuran-3(2H)-one (composé **I29,** tableau 3)
(Z)-2-(4-fluorobenzylidène)-4,6-diméthoxy-7-(1-méthyl-1,2,3,6-tétrahydropyridin-4-yl)benzofuran-3(2H)-one (composé **I30)**
(*Z*)-2-(2,6-dichlorobenzylidène)-4,6-diméthoxy-7-(1-méthyl-1,2,3, 6-tétrahydropyridin-4-yl)benzofuran-3(2*H*)-one (composé **I31)**
(*Z*)-2-benzylidène-4,6-diméthoxy-7-(1-méthyl-1,2,3,6-tétrahydropyridin-4-yl)benzofuran-3(2H)-one (composé **I32)**
(*E*)-4,6-diméthoxy-7-(1-méthyl-1,2,3,6-tétrahydropyridin-4-yl)-2-(naphtalèn-1-ylméthylène)benzofuran-3(2*H*)-one (composé **I33)**
(*Z*)-2-(furan-2-ylméthylène)-4,6-diméthoxy-7-(1-méthyl-1,2,3,6-tétrahydropyridin-4-yl)benzofuran-3(2H)-one (composé **I34)**
(*E*)-4,6-diméthoxy-7-(1-méthyl-1,2,3,6-tétrahydropyridin-4-yl)-2-(thiophèn-2-ylméthylène)benzofuran-3(2H)-one (composé **I35)**
(*E*)-2-(2-chlorobenzylidène)-7-(3-hydroxy-1-méthyl-pipéridin-4-yl)-4,6-diméthoxy-benzofuran-3(2H)-one (composé I36)
(*Z*)-2-(furan-2-ylméthylène)-4-hydroxy-6-méthoxy-7-(1-méthylpipéridin-4-yl)benzofuran-3(2H)-one (composé **I37)**
(*E*)-4-éthoxy-2-(2-fluorobenzylidène)-7-(1-méthyl-1,2,3,6-tétrahydropyridin-4-yl)-benzofuran-3(2H)-one (composé **I38).**

3. Composé
(*Z*)-2-(3-bromobenzylidène)-4,6-diméthoxy-7-(1-méthyl-pipéridin-4-yl)-benzofuran-3(2H)-one (composé **I10)** ;
(*Z*)-2-(3-bromo-4-fluorobenzylidène)-4,6-diméthoxy-7-(1-méthylpipéridin-4-yl)-benzofuran-3(2H)-one (composé **I11)** ;
(*E*)-4,6-dihydroxy-7-(1-méthyl-pipéridin-4-yl)-2-(thiophèn-2-ylméthylène)-benzofuran-3(2H)-one (composé **I24)** ;
(*Z*)-2-(4-bromobenzylidène)-4-éthoxy-7-(1-méthyl-pipéridin-4-yl)-benzofuran-3(2H)-one (composé **I26)** ; et
(*Z*)-2-(4-bromobenzylidène)-4-éthoxy-7-(1-méthyl-1,2,3,6-tétrahydropyridin-4-yl)-benzofuran-3(2H)-one (composé **I39)**
ou sels pharmaceutiquement acceptables de ceux-ci.

4. Composé selon la revendication 1 ou la revendication 3 destiné à être utilisé dans le traitement de la tuberculose.

5. Composé selon la revendication 1 ou la revendication 3, dans lequel les sels pharmaceutiquement acceptables sont des sels d'un acide choisi dans le groupe constitué d'acide chlorhydrique, acide sulfurique, acide phosphorique, acide acétique, acide citrique, acide oxalique, acide malonique, acide salicylique, acide malique, acide fumarique, acide succinique, acide ascorbique, acide maléique, acide méthanesulfonique et acides isoéthoniques.

6. Procédé de préparation de composés selon la revendication 1 et la revendication 3, dans lequel les étapes de procédé comprennent :
(i) la réaction du composé de formule 2 ou 3 avec un composé aldéhyde (formule 4) en présence d'une base dans un solvant protique à une température comprise entre 20 et 100 °C pendant une durée dans la plage de 2 à 12 h pour obtenir le composé de formule 6 ou 5, respectivement, 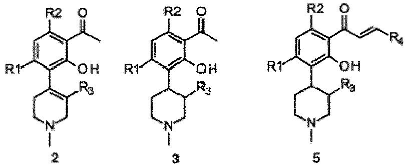 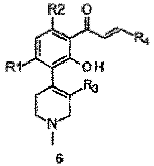
(ii) la réaction du composé de formule 5 ou 6 avec Hg(OAc)₂ en présence d'une base à une température dans la plage de 60 à 150 °C pendant une durée dans la plage de 1 à 12 h pour obtenir le composé de formule I ou l'un quelconque des composés I10, I11, I24, I26 ou I39 selon la revendication 3.

7. Procédé selon la revendication 6, dans lequel l'aldéhyde est choisi dans un groupe constitué d'alkyle, alkyle substitué, styryle, styryle substitué, aryle, aryle substitué, biaryle, biaryle substitué, naphtyle, anthracényle, hétérocycles ou hétéroaryle substitués ou non substitués et hétérocycle ou hétéroaryle est choisi dans le groupe constitué de furanyle, thiophényle, pérazinyle, morpholinyle, pipéridyle, pyridyle, triazolyle, triazinyle, pyrimidinyle, pyridazinyle, oxazolyle, benzofuranyle, pyrrolyle, imidazolyle, thiazoyle, quinolinyle, isoquinolinyle, benzooxazolyle, benzothiazolyle et en outre aryle substitué, styryle substitué, vinyle substitué, biaryle substitué et hétéroaryle ou hétérocycle substitué, dans lesquels la substitution va de 1 à 4 et les substituants sont choisis indépendamment dans le groupe constitué de F, Cl, Br, I, NR_{I1}R_{I2}, CF₃, OCF₃, OR₁₃, NO₂, chaîne alkyle de C₁ à C₁₄,
R_{I1}, R_{I2}, et R_{I3} sont chacun choisis indépendamment dans le groupe constitué de H, alkyle, alkylphényle substitué, phényle substitué.

8. Procédé selon la revendication 6, dans lequel la base utilisée à l'étape i) est choisie dans un groupe constitué de KOH, NaOH, K₂CO₃, Cs₂CO₃.

9. Procédé selon la revendication 6, dans lequel la base utilisée à l'étape ii) est choisie dans un groupe constitué de pyridine ou triéthylamine.

10. Procédé selon la revendication 6, dans lequel le solvant protique utilisé est choisi dans un groupe constitué de méthanol, éthanol et eau.

11. Composition pharmaceutique comprenant le composé de formule 1 selon la revendication 1 ou les composés I10 ou I11 ou I24 ou I26 ou I39 selon la revendication 3, éventuellement conjointement avec un support, un sel, des excipients ou diluants pharmaceutiquement acceptables.
